# EUROPEAN PATENT SPECIFICATION

(11) **EP 2 777 616 B1**
(45) Date of publication and mention of the grant of the patent: **19.08.2020**
(21) Application number: 14159271.7
(22) Date of filing: 12.03.2014
(51) Int. Cl.: A61F 2/24

(54) **Prosthesis for atraumatically grasping intralumenal tissue**
Prothese zum atraumatischen Ergreifen von intraluminalem Gewebe
Prothèse pour saisie atraumatique de tissus intracavitaires

(30) Priority: 14.03.2013 US 201361782707 P; 15.03.2013 US 201361798115 P
(43) Date of publication of application: 17.09.2014
(73) Proprietor: Edwards Lifesciences CardiAQ LLC, Irvine, CA 92614 (US)
(72) Inventor: Ratz, J. Brent, Irvine, California 92618 (US); Quadri, Arshad, Irvine, California 92618 (US); Pesce, Luca, Irvine, California 92618 (US)
(74) Representative: Drees, Erika Carol

(56) References cited:
- WO-A2-2012/177942
- CA-A1- 2 827 556
- US-A1- 2011 137 397
- US-A1- 2012 078 353
- US-A1- 2012 078 360

## Description

### BACKGROUND OF THE INVENTION

### Field of the Invention

Certain embodiments disclosed herein relate generally to prostheses for implantation within a lumen or body cavity. In particular, certain embodiments relate to expandable prostheses such as replacement heart valves, such as for the mitral valve, that are configured to atraumatically grasp intralumenal tissue.

### Description of the Related Art

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner. Further challenges arise when trying to controllably deliver and secure such prostheses in a location such as at a native mitral valve. These replacement valves are often intended to at least partially block blood flow. However, a problem occurs when blood flows around the valve on the outside of the prosthesis. For example, in the context of replacement heart valves, paravalvular leakage has proven particularly challenging.

CA 2 827 556 A1 discloses an annular body formed from a wire mesh, arm-shaped ventricular anchoring elements which form an angle with the outer wall and transition to rings, and atrial anchoring elements which transition to rings. The ventricular and atrial anchoring elements are bent once so that each element extends along a single segment up to the respective ring.

US 2011/0137397 A1 discloses a frame comprising a tubular main body, one or more ventricular anchors and optionally an atrial sealing member.

US 2012/0078360 A1 discloses a valve prosthesis, a proximal portion including one or more fastening, anchoring or bracing mechanisms, and a distal portion including one or more fastening, anchoring or bracing mechanisms.

WO 2012/177942 A2 discloses a support including a plurality of fingers and arms.

US 2012/0078353 A1 describes a replacement heart valve that includes a body and a frame. A portion of the frame is configured to foreshorten, such that the frame longitudinally expands when the frame is urged to a radially compacted state and longitudinally contracts when the frame is urged to a radially expanded state.

### SUMMARY OF THE INVENTION

Embodiments of the present disclosure are directed to a prosthesis, such as but not limited to a replacement heart valve. A prosthesis according to the invention includes the features of claim 1. In some embodiments, a replacement heart valve for a native heart valve, such as a mitral valve, are provided. In the following disclosure, the use of the expression "embodiment" or the indication of optional features should not be construed to mean that protection is sought beyond the scope of protection defined by the claims.

In some embodiments, a prosthesis can comprise an expandable frame, and a plurality of anchors, for example a plurality of proximal anchors and a plurality of distal anchors. The expandable frame may comprise a proximal end and a distal end and having a longitudinal axis extending between the proximal end and the distal end. The anchors can extend outwardly from the frame. The frame can be configured to radially expand and contract for deployment within the body cavity.

In some embodiments, the frame comprises a plurality of foreshortening cells causing a longitudinal length of the frame to decrease as the frame moves from a collapsed size to an expanded size. In some embodiments, proximal anchors and distal anchors may be separated by a foreshortening portion of the frame. In some embodiments, some of the plurality of anchors may be connected to a non-foreshortening portion of the frame, and some of the plurality of anchors are connected to a foreshortening portion of the frame.

In some embodiments, when the frame is in an expanded configuration, the frame can have a larger cross-sectional dimension in a middle portion of the frame and a smaller cross-sectional dimension in a proximal portion and a distal portion of the frame, wherein the middle portion is between the proximal and distal portions. In other embodiments, the frame may have a cylindrical or substantially cylindrical shape when in an expanded configuration, wherein the proximal end and the distal end of the frame have the same or substantially the same cross-sectional dimension.

In some embodiments, at least some of the anchors, or alternatively all of the anchors, comprise a loop that forms an atraumatic end of a corresponding anchor. For example, proximal anchors and/or distal anchors may comprise looped ends. In some embodiments, the anchors having looped ends may connect to the frame at spaced apart base locations. For example, the spaced apart locations may be at opposite corners of the same cell of a frame. Additionally or alternatively, at least some anchors which may or may not have looped ends may connect to the frame with a single strut. Adjacent ends of anchors may be circumferentially spaced apart by one or more cells of the frame. In some embodiments, when the frame is in an expanded configuration, a width of each of the plurality of anchors having looped ends as defined circumferentially around the longitudinal axis over the entire length of the anchor from the base to the looped end is less than the width of one cell of the frame. In some embodiments, the looped ends of each anchor may be circumferentially aligned with the base where the anchor connects to the frame so that the looped end and the base are both located within a common plane that is parallel to and intersects the longitudinal axis.

In some embodiments, the base of each of the plurality of proximal and/or distal anchors is at a location where corners of two adjacent cells of the frame meet. In some embodiments, the base of each of the plurality of distal anchors is at a distalmost corner of a cell. In some embodiments, the base of each of the plurality of proximal anchors is at a proximalmost corner of a cell. In some embodiments, the ends of the plurality of distal anchors are not circumferentially aligned with the ends of the plurality proximal anchors when the frame is in an expanded configuration.

A prosthesis according to certain embodiments can comprise a plurality of proximal anchors connected to the frame so that when the frame is in an expanded configuration an end of each proximal anchor is positioned radially outward from the frame and extends generally distally. Alternatively or in addition, a plurality of distal anchors may be connected to the frame so that when the frame is in an expanded configuration an end of each distal anchor is positioned radially outward from the frame and extends generally proximally. The ends of the distal anchors may be axially spaced from the ends of the proximal anchors when the frame is in an expanded configuration. In some embodiments, the frame is configured such that radial expansion of the frame causes the ends of the plurality of proximal anchors and the ends of the plurality of distal anchors to draw closer together.

In some embodiments, a plurality of proximal anchors may extend from the frame and be expandable from a collapsed configuration to an expanded configuration, wherein in the expanded configuration, each proximal anchor extends proximally away from the proximal end of the frame and then turns to extend distally, each proximal anchor extending to an end positioned radially outward from the frame. The plurality of proximal anchors may in some embodiments extend from or a near the proximal end of the frame. The ends of the proximal anchors may be distal to the proximal end of the frame or distal to where the proximal anchors connect to the frame when the frame is at least partially expanded and the proximal anchors are in their expanded configuration.

In some embodiments, a plurality of distal anchors may extend from the frame and be expandable from a collapsed configuration to an expanded configuration, wherein in the expanded configuration, each distal anchor extends distally away from the distal end of the frame and then turns to extend proximally, each distal anchor extending to an end positioned radially outward from the frame. The plurality of distal anchors may in some embodiments extend from or a near the distal end of the frame. The ends of the distal anchors may be proximal to the distal end of the frame or distal to where the distal anchors connect to the frame when the frame is at least partially expanded and the distal anchors are in their expanded configuration.

In some embodiments, the frame and the plurality of anchors (for example the plurality of proximal anchors and the plurality of distal anchors) are formed from a single piece of material. For example, the frame, the plurality of proximal anchors and the plurality of distal anchors may be formed by laser cutting a metal tube.

In some embodiments, the ends of at least some of the proximal anchors and/or distal anchors are substantially parallel with the longitudinal axis when the frame is at least partially expanded and the proximal and distal anchors are in their expanded configurations. Alternatively or additionally, the ends of at least some of the proximal anchors and/or distal anchors may be substantially perpendicular with the longitudinal axis when the frame is at least partially expanded and the proximal and distal anchors are in their expanded configurations. Alternatively or additionally, the ends of at least some of the proximal anchors and/or distal anchors may be inclined with respect to the longitudinal axis when the frame is at least partially expanded and the proximal and distal anchors are in their expanded configurations.

In some embodiments, some of the anchors expand to a first radial distance, and some of the anchors expand to a second radial distance, the first radial distance being greater than the second radial distance. For example, at least some of the plurality of proximal anchors may expand to a first radial distance, and at least some of the plurality of distal anchors may expand to a second radial distance, the first radial distance being greater than the second radial distance. Alternatively, at least some of the plurality of distal anchors may expand to a first radial distance, and at least some of the plurality of proximal anchors may expand to a second radial distance, the first radial distance being greater than the second radial distance.

Some embodiments of the prosthesis further comprise a valve body attached to the expandable frame. A replacement mitral valve may also be provided that comprises any of the prostheses described herein. The frame of a prosthesis may be positionable within a native mitral valve such that proximal anchors atraumatically engage tissue on an atrial side of the native mitral valve annulus, and distal anchors are positionable within the ventricle and have portions that extend generally proximally toward a ventricular side of the native mitral valve annulus. The distal anchors may be positionable within the ventricle and extend proximally to ends spaced distally from the native mitral valve annulus. Alternatively, the distal anchors may be positionable within the ventricle and extend proximally to ends that engage the native mitral valve annulus.

In some embodiments, the frame may be configured so that it does not apply significant radial force to the native valve annulus to thereby permit the frame to axially reciprocate relative to the native mitral valve annulus. In some embodiments, at least some of the plurality of anchors are configured to expand to a radial distance from a central longitudinal axis of the frame that is about 150% or more of a radius of the frame when the frame is in an expanded configuration. In some embodiments, at least some of the plurality of anchors are configured to expand to a radial distance from the central longitudinal axis that is about 180% or more of the radius of the frame when the frame is in an expanded configuration.

In some embodiments, a prosthesis may further comprise an outer skirt surrounding the frame. The outer skirt may be connected to or near at least some of the ends of the anchors (e.g., the proximal anchors) and configured to move radially outward with the anchors when the anchors move from a collapsed configuration to an expanded configuration. The outer skirt is configured to prevent paravalvular leakage exterior to the frame when the anchors are in an expanded configuration.

Methods of delivering a prosthesis and/or securing the prosthesis to intralumenal tissue, and use of a prosthesis to secure to intraluminal tissue, for example as a replacement heart valve to secure to a mitral valve or other annulus, are described for the purpose of understanding the present disclosure. For example, a method of delivering a replacement valve to a native mitral valve can comprise one or more of the following steps. Delivering a replacement valve mounted on a delivery device to the native mitral valve annulus while the replacement valve is in a radially compacted state, the replacement valve comprising a radially expandable frame comprising a proximal end, a distal end, a plurality of distal anchors extending generally proximally from the frame, and a plurality of proximal anchors extending generally distally from the frame. Positioning the replacement valve so that ends of the distal anchors are on a ventricular side of the native leaflets beyond a location where chordae tendineae connect to free ends of the native leaflets. Releasing at least a portion of the replacement valve from the delivery device to thereby expand the distal anchors radially outwardly to a first radial dimension. Moving the ends of the distal anchors toward the ventricular side of the native valve annulus with the distal anchors extending between at least some of the chordae tendineae to provide tension on the chordae tendineae. Further releasing the replacement valve from the delivery device to thereby expand the proximal anchors radially outwardly to a second radial dimension greater than the first radial dimension, wherein the proximal anchors upon further release of the replacement valve from the delivery device move into engagement with tissue on an atrial side of the native valve annulus while the distal anchors provide tension on the chordae tendineae.

In some embodiments a prosthesis can comprise an expandable frame, a plurality of distal anchors and a plurality of proximal anchors. The anchors can extend outwardly from the frame. The frame can be configured to radially expand and contract for deployment within the body cavity. When the frame is in an expanded configuration, the proximal anchors can extend a significant distance away from the exterior of the frame, such as a length of about one-half or more the diameter of the frame. In some embodiments, at least some of the anchors comprise a loop that forms an atraumatic end of a corresponding anchor. In some embodiments, an outer skirt may be positioned annularly around an exterior of the expandable frame and be connected to some of the anchors to create an axial barrier to fluid flow exterior to the frame when deployed within the body cavity.

The following disclosure also describes methods of delivering a prosthesis, e.g. a replacement heart valve, methods of securing a prosthesis to intralumenal tissue, and methods of using a prosthesis to create a barrier to fluid flow exterior to the prosthesis (e.g., to prevent paravalvular leakage).

In some embodiments, a prosthesis can be configured to grasp intralumenal tissue when deployed within a body cavity. The prosthesis can comprise an expandable frame, a plurality of proximal anchors connected to the frame, and a plurality of distal anchors connected to the frame. The expandable frame can comprise a proximal end and a distal end and a longitudinal axis extending therethrough, the frame configured to collapse radially for delivery and to expand radially upon deployment. The plurality of proximal anchors can be expandable to a configuration wherein a portion of each of the proximal anchors extends generally distally and an end of each of the proximal anchors is positioned radially outward from the frame. The plurality of distal anchors can be expandable to a configuration wherein a portion of each of the distal anchors extends generally proximally and an end of each of the distal anchors is positioned radially outward from the frame. Expansion of the frame from a first at least partially collapsed size to a second expanded size can cause the ends of the proximal anchors and the ends of the distal anchors to draw closer together. At least some of the plurality of proximal anchors can be configured to expand to a radial distance from a central longitudinal axis of the frame that is about 150% or more of a radius of the frame when the frame is in an expanded configuration.

### BRIEF DESCRIPTION OF THE DRAWINGS

These and other features, aspects and advantages are described below with reference to the drawings, which are intended to illustrate but not to limit the invention. In the drawings, like reference characters denote corresponding features consistently throughout similar embodiments.
Figure 1A is a side view of a frame for a prosthesis.
Figure 1B is a top perspective view of the frame of Figure 1A where the anchors have not yet been bent to the desired shape.
Figure 2 is an embodiment of a prosthesis configured as a replacement heart valve.
Figure 3 is an embodiment of a prosthesis configured as a replacement heart valve.
Figure 4 is an embodiment of a prosthesis configured as a replacement heart valve.
Figure 5 is a schematic representation of a prosthesis positioned within the heart.
Figure 6 is a detail schematic representation of the prosthesis positioned within the heart of Figure 5.
Figure 7A is a side view of an embodiment of a prosthesis.
Figure 7B is a flat pattern view of the prosthesis of Figure 7A.
Figure 8A is a side view of an embodiment of a prosthesis.
Figure 8B is a flat pattern view of the prosthesis of Figure 8A.
Figure 9A is a side view of an embodiment of a prosthesis.
Figure 9B is a flat pattern view of the prosthesis of Figure 9A.
Figure 10A is a side view of an embodiment of a prosthesis.
Figure 10B is a side view of the prosthesis of Figure 10A.
Figure 11 is a side view of an embodiment of a prosthesis.
Figure 12A is a side view of an embodiment of a prosthesis configured as a replacement heart valve.
Figure 12B is a side view of the prosthesis of Figure 12A.
Figure 12C is a bottom view of a prosthesis similar to the embodiment of Figure 6A.
Figure 12D is a bottom view of the prosthesis of Figure 12B.
Figure 13A is a side view of an embodiment of a prosthesis configured as a replacement heart valve.
Figure 13B is a side view of the prosthesis of Figure 13A.
Figure 14A is a side view of an embodiment of a prosthesis.
Figure 14B is a side view of the prosthesis of Figure 14A.
Figure 14C is a detail view of a portion of the prosthesis of Figure 14A.
Figure 14D is a detail view of a portion of the prosthesis of Figure 14A.
Figure 15A is a side view of an embodiment of a prosthesis.
Figure 15B is a side view of the prosthesis of Figure 15A.
Figure 15C is a bottom view of the prosthesis of Figure 15A, configured as a replacement heart valve.
Figure 15D is a bottom view of the prosthesis of Figure 15A, configured as a replacement heart valve.
Figure 16A is a schematic representation of a prosthesis positioned within the heart.
Figure 16B is a detail schematic representation of the prosthesis positioned within the heart of Figure 16A.

### DETAILED DESCRIPTION OF THE PREFERRED EMBODIMENTS

The present specification and drawings provide aspects and features of the disclosure in the context of several embodiments of prostheses, replacement heart valves, delivery devices and methods that are configured for use in the vasculature of a patient, such as for replacement of natural heart valves in a patient. These embodiments may be discussed in connection with replacing specific valves such as the patient's aortic or mitral valve. However, it is to be understood that the features and concepts discussed herein can be applied to products other than heart valve implants. For example, the controlled positioning, deployment, and securing features described herein can be applied to medical implants, for example other types of expandable prostheses, for use elsewhere in the body, such as within a vein, or the like. In addition, particular features of a valve, delivery device, etc. should not be taken as limiting, and features of any one embodiment discussed herein can be combined with features of other embodiments as desired and when appropriate.

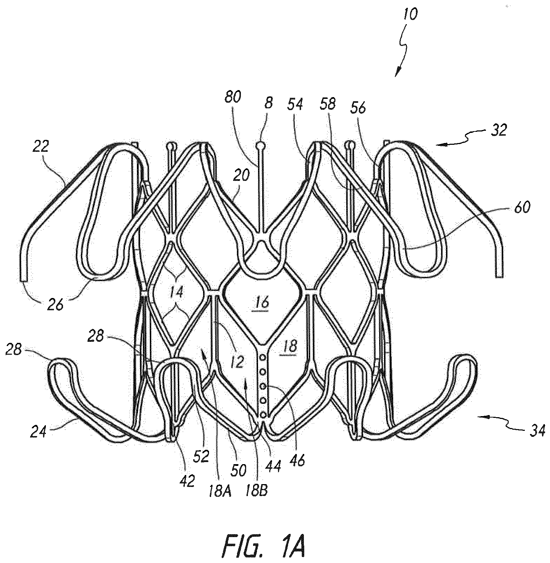

With initial reference to Figures 1A-B, an embodiment of a prosthesis 10 is shown. The illustrated prosthesis 10 includes a frame 20 that may be self-expanding or balloon expandable. The frame 20 can include a proximal end 32, a distal end 34 and proximal 22 and distal 24 anchors. The anchors can allow the frame to engage a native valve annulus or other tissue to be implanted at a target location. The prosthesis 10 can include one or more of a valve 60, an outer skirt 30, and a valve skirt 70 as will be described in more detail below. The valve 60 can be designed to replace a damaged or diseased native heart valve such as a mitral valve; though it will be understood that a replacement valve is not required as part of the prosthesis.

The prosthesis can be a replacement heart valve similar to that and including features similar to those disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931, 2011/0313515 and 2012/0078353.

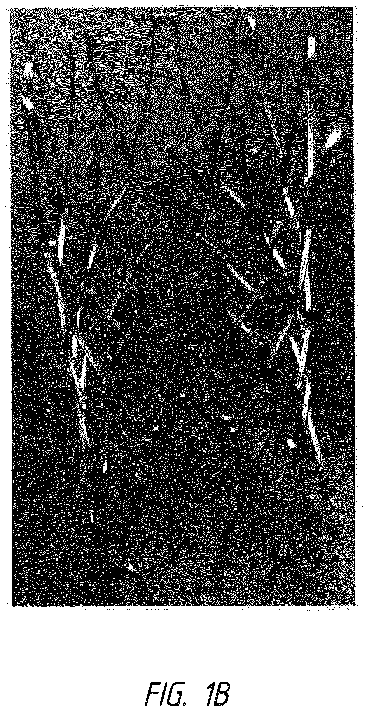

The frame 20 can be made of many different materials, but is preferably made from metal. In some embodiments, the frame 20 can be made from a shape memory material, such as nitinol. A wire frame or a metal tube can be used to make the frame. The wire frame of a metal tube can be cut or etched to remove all but the desired metal skeleton. In some embodiments a metal tube is laser cut in a repeating pattern to form the frame. The flat pattern can be cut from a metal tube and then the tube can be bent and expanded to the shape shown in Figures 1A-B. The frame 20 can further be expanded and/or compressed and/or otherwise worked to have the desired shape or shapes, such as for introduction and implantation. Figure 1B shows the frame 20 in a partially finished state. The frame has been cut and initially expanded, but the anchors 22, 24 have not yet been bent as shown in Figure 1A.

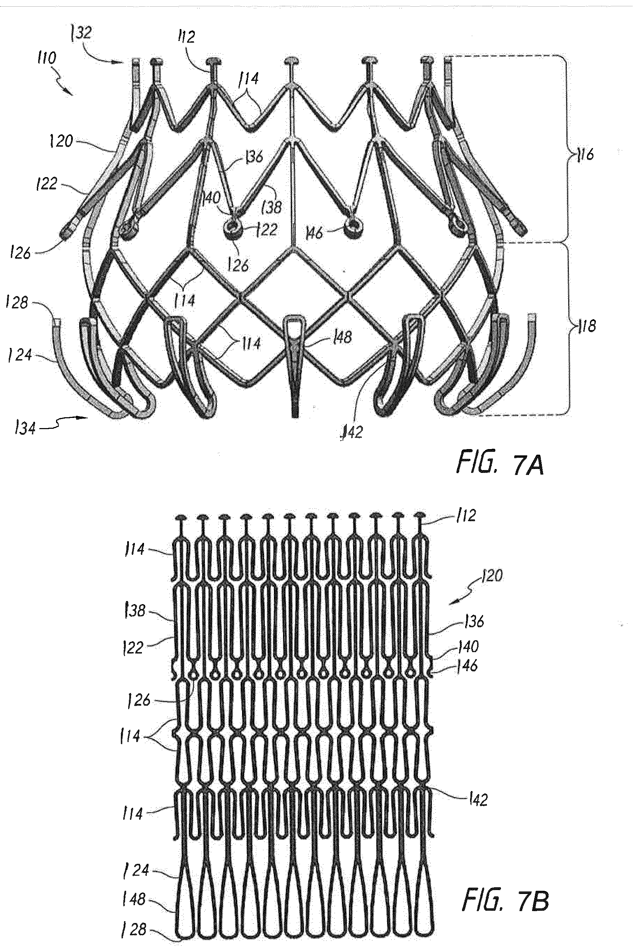

As shown, the frame when in an expanded configuration, such as in a fully expanded configuration, has a cylindrical or slightly cylindrical shape, where a middle portion is substantially similar in shape and size as the proximal 32 and distal 34 ends. The frame can be a substantially cylindrical shape with the same or substantially the constant cross-sectional dimension or diameter from the proximal end to the distal end. The cylindrical shape of the frame, in combination with the anchors described below, can advantageously allow the frame to float within a native valve while the anchors engage a native valve annulus or other body cavity and spacing the inlet and outlet of the frame away from the heart or vessel wall. This can help reduce undesired contact between the prosthesis and the heart or vessel, such as the ventricular wall of the heart. The prosthesis 10 and frame 20 may be similar to the replacement heart valves and associated frames disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931 and 2011/0313515. Further examples of frame and prosthesis configurations that may also be utilized are described with respect to Figures 7A-16B.

A number of struts collectively make up the frame 20. Figure 1A illustrates the frame in an expanded configuration with a number of longitudinal struts 12 and undulating struts 14, with cells 16, 18 defined by the open spaces between the struts. The longitudinal struts may be arranged so that they are parallel or generally or substantially parallel to a longitudinal axis of the frame. The longitudinal axis of the frame may be defined as the central axis that extends through the center of the frame between the proximal 32 and distal 34 ends. Any number of configurations of struts can be used, such as the rings of undulating struts shown forming chevrons and diamonds, but also ovals, curves, and various other shapes. The illustrated embodiment includes two rows of diamond-shaped cells 16 at the top or adjacent the proximal end and then a row of sideways diamond-shaped cells 18 at the bottom or near the distal end. These sideways diamond-shapes are made of two longitudinal struts 12 that are offset from one another, or one is higher than the other, and then an undulating strut 14 connects the longitudinal struts 12 at the top and bottom of the cell. The sideways diamond-shapes alternate between sides as to which side is higher and which is lower. So a first sideways diamond-shaped cell 18A has a low left side and a high right side, and a second sideways diamond-shaped 18B had a high left side and a low right side, where the high right side of cell 18A shares the same longitudinal strut 12 as the high left side of cell 18B.

Some of the struts can include one or more eyelet 46. As illustrated, a plurality of eyelets, here five, are located along one of the longitudinal struts 12 as part of the sideways diamond-shaped cells. One or more eyelets can be positioned in other locations along the frame and/or anchors. The eyelets 46 may be used to attach features such as the valve 60, outer skirt 30, and/or valve skirt 70 to the frame 20.

The frame 20 as illustrated is foreshortening. The foreshortening can be defined by the frame 20 and the positioning of various types of struts along the frame 20. When the frame is radially collapsed or compacted, the struts 14 become more parallel with respect to the longitudinal axis of the frame, causing cells 16 and 18 to collapse, causing an outer diameter of the frame to decrease and the longitudinal length of the frame to increase. As the frame moves from a compacted position to an expanded position, the cells 16 and 18 widen sideways and the longitudinal length of the frame can decrease. It will be appreciated while in some embodiments the entire length of the frame 20 is foreshortening, in other embodiments such as embodiments of the prostheses described in the patents and applications mentioned above, only a portion of the frame is foreshortening.

Foreshortening of the frame 20 can be used to engage and secure the prosthesis to intralumenal tissue in a body cavity, for example tissue at or adjacent a native valve, such as a native valve annulus and/or leaflets. Opposing anchors 22, 24 can be constructed on the frame 20 so that portions of the anchors, such as tips or ends 26, 28, move closer together as the frame foreshortens. As one example, this can allow the anchors 22, 24 to grasp tissue on opposite sides of the native mitral annulus to thereby secure the prosthesis at the mitral valve.

The anchors 22, 24 and anchor tips 26, 28 can be located anywhere along the frame 20 just so long as at least one of the anchors is either connected to a foreshortening portion or a foreshortening portion is positioned between the anchors so that a portion of the anchors will be move closer together with expansion of the frame. As shown in Figure 1A, the foreshortening portion extends the entire or substantially the entire length of the frame.

Preferably, each of the anchors 22, 24 is positioned or extends generally radially outwardly from the frame 20 so that the anchor tips 26, 28 are generally spaced away or radially outward from the rest of the frame 20. For example, the anchor tips may be located radially outward from the middle portion of the frame, with the tips 26 and 28 being axially spaced from one another. In some embodiments, all or part of the structure connected to the anchor tip and extending radially from the frame, including one or more rings and/or struts, can be considered part of the anchor. The anchors can include a base located on the anchor on a side opposite the tip. The base can be for example where the anchor begins to extend from or away from the frame 20.

For example, the distal anchors 24 are shown having looped anchors. Each looped anchor has a first base 42 and a second base 44 connected to the frame, wherein the first and second bases are at the distal-most corners of adjacent cells. Alternatively, the first and second bases may be located at adjacent corners of the same cell, or at opposite corners of the same cell if for example the cells adjacent the distal end of the frame have the same configuration as the cells shown in Figure 1A at the proximal end of the frame. The distal anchors 24 extend generally distally away from the frame and are bent near the base 42 to extend radially outward away from the frame along a first segment 50. Then the anchor is bent to point proximally or generally proximally along a second segment 52 ending in the tip 28. The tips 28 of the distal anchors may be curved or arcuate atraumatic tips. The ends of the distal anchors can extend proximally and be parallel or substantially parallel with the longitudinal axis of the frame, or they may extend generally proximally but still radially outwardly inclined or at an acute angle relative to the longitudinal axis of the frame. The distal anchor 24 then repeats this configuration in reverse towards the second base 44 such that the two sides of the looped anchor are mirror images of one another.

In addition, as illustrated in Figure 1A, adjacent distal anchors share one strut so that each distal anchor is directly adjacent another distal anchor. In other embodiments, distal anchors can be spaced apart, for example with at least a cell 18 or two cells 18 located between the distal anchors. It will be understood that the distal anchors can have other configurations such as described in the applications mentioned above, and for example as shown in Figures 7A-16B, and that the distal anchors may not be symmetrical.

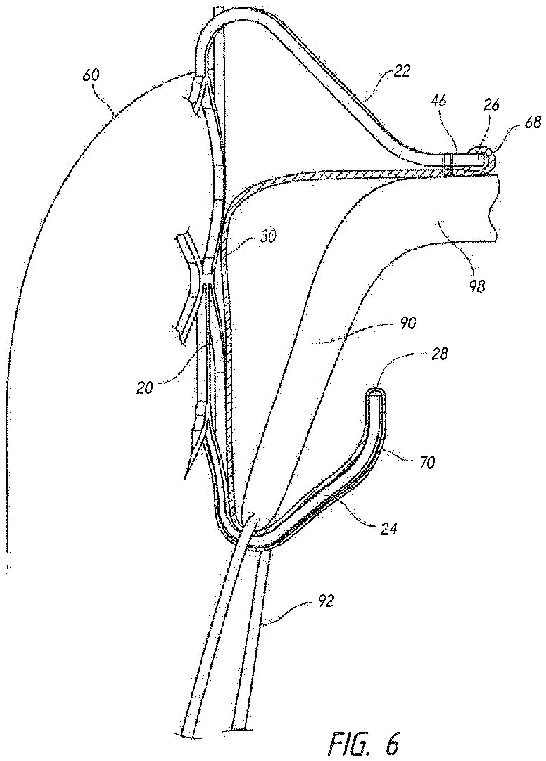

The proximal anchors 22 are shown having looped anchors of a similar shape and configuration as the distal anchors 24. It can be seen that each proximal anchor extends from or near the proximal end of the frame at bases 54 and 56 located on adjacent proximal-most corners of cells 16. In the embodiment illustrated in Figure 1A, the proximal anchors are longer than the distal anchors, and as such, the proximal anchors may extend further away from the frame. As shown the anchors extend proximally at the bases 54, 56 and are then bent to segment 58 which extends radially outwardly away from the frame in a generally distal direction. Then the proximal anchor is bent to point distally or generally distally along a second segment 60 ending in the tip 26. The tips 26 of the proximal anchors may be curved or arcuate atraumatic tips. The ends of the proximal anchors can extend distally and be parallel or substantially parallel with the longitudinal axis of the frame, or they may extend generally distally but still radially outwardly inclined or at an acute angle relative to the longitudinal axis of the frame. Another embodiment, described below with respect to Figure 6, has the ends of the proximal anchors becoming perpendicular to the longitudinal axis of the frame.

In addition, adjacent proximal anchors can share the same base where the anchors are bent outward from the frame, or may be considered to extend from the same corner on cell 16. In other embodiments, proximal anchors can be spaced apart so that there is at least a ½ cell or one cell located between the proximal anchors. It will be understood that the proximal anchors can have other configurations such as described in the applications incorporated by reference and for example, as shown in Figures 7A-16B, and that the proximal anchors may not be symmetrical. As illustrated in Figure 1A, between the bases 54 and 56 of each proximal anchor, there may also be located a longitudinally extending strut 80 extending proximally, e.g. from the proximal-most corner of a cell 16 in the second row of cells from the proximal end. These struts 80 may terminate at their proximal ends in an enlarged portion such as a tab 8 that may facilitate holding or retaining the proximal end of the frame, such as in a delivery system as described below.

In some embodiments, in an expanded state such as shown in Figure 1A, at least some of the proximal anchors can extend to a radial distance from an exterior surface of the frame that is ½ (or about ½) or more of the expanded diameter of the frame. In some embodiments, all of the proximal anchors extend at least to this radial distance. In even further embodiments, all of the proximal and distal anchors extend at least to this radial distance. In other embodiments, the radial distance of one or more of the ends of the anchors from a central longitudinal axis passing through the middle of the frame may be 150% (or about 150%) or more, 180% (or about 180%) or more, 200% (or about 200%) or more, 220% (or about 220%) or more, or 250% (or about 250%) or more of the radius of the frame when the frame and the anchors are in expanded configurations. For example, if the radius of the frame is 16 mm and a proximal anchor end is spaced 9 mm from the exterior of the frame, that proximal anchor extends 25 mm from the central longitudinal axis of the frame, and is 156.25% of the radius of the frame.

In some embodiments the diameter of the frame 20 may be in the range of 20-40 mm (or about 20 to about 40 mm), more preferably 25-35 mm (or about 25 to about 35 mm) when expanded. The outermost tip diameter may be greater than the frame diameter as described above and may be in the range of 40-60 mm (or about 40 to about 60 mm), and in some embodiments may be about 50 mm when the frame diameter is about 30 mm. In some embodiments the length of the prosthesis, from proximal to distal end, when compressed, is between 20-40 mm (or about 20 to about 40 mm), more preferably 25 to 30 mm (or about 25 to about 30 mm), for example about 29 mm. When expanded, the prosthesis may have a length between 15 to 20 mm (or about 15 to 20 mm), more preferably 17 to 18 mm (or about 17 to about 18 mm).

The distal anchors 24 can be positioned to be not as far radially outward as the proximal anchors, and the tips 28 may be positioned radially inward of the tips 26. As described further below, such a configuration may be advantageous in positioning and securing the prosthesis in a mitral valve or other body location. In some embodiments, as illustrated in Figure 1A, the ends or tips 26 of the proximal anchors 22 are positioned further radially outward from the frame 20 than the ends of tips 28 distal anchors 24 when the frame and the anchors are in an expanded configuration, e.g., when they are fully expanded. In other embodiments, the distal anchors and proximal anchors can be positioned at the same radial outward dimension, or the distal anchors may even be positioned further outward than the proximal anchors. In further embodiments, some of the proximal anchors (or distal anchors) may extend to a first radial distance, and others of the proximal anchors (or distal anchors) may extend to a second radial distance, where the first radial distance is greater than the second radial distance. The distal anchors in Figure 1A are shown to be circumferentially staggered with respect to the proximal anchors, meaning that the tips 26 of the proximal anchors are not aligned, and are circumferentially in between the tips 28 of the distal anchors. In other embodiments, the tips 26 and 28 may be circumferentially aligned.

It will be understood that the anchors can have various other configurations. In some embodiments, each of the anchors can extend radially outwardly from the frame at an anchor base and terminate at an anchor tip. The anchors can be connected to the frame at one of many different locations including apices, junctions, other parts of struts, etc. The anchors can comprise first, second, third, or more spaced apart bending stages along the length of each anchor. The anchors can also extend either distally or proximally before and/or after one or more of the bending stages. A portion of the anchor may extend with the frame before or after any bending stages. As shown, the anchors 22, 24 may comprise loops as described above, having a curved or arcuate atraumatic tip to minimize damage to body tissue. Further details that may be incorporated and/or interchanged with the features described herein are disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931, 2011/0313515 and 2012/0078353. For example, the frame 20 and/or the anchors 22, 24 may have configurations as shown with respect to Figures 7A-16B.

As shown in Figure 1B, in one embodiment the prosthesis has nine distal anchors and nine proximal anchors. Any number of proximal and distal anchors may be used. In other embodiments, instead of a 1:1 correspondence between anchors, other ratios, such as a 9:6 or a 9:3 correspondence between the anchors, are possible.

With respect to the number of cells and rows of cells, when there are nine proximal anchors and nine distal anchors, there may be two rows of nine cells 16 each. The cells in the second row can share struts 14 from the first row. A third row at the distal end can have a different cell configuration with sideways diamonds as illustrated in Figure 1A, and can for example have eighteen cells 18, with each cell 18 sharing a strut from a cell in the second row. In other embodiments, the third row can include cells similar in shape to cells 16 in the first and second rows. In another embodiment, a frame may have one or more rows of diamond-shaped cells, where the number of cells per row is 12 or some other number.

The anchor tips 26 and 28 as described above advantageously provide atraumatic surfaces that may be used to grasp intralumenal tissue without causing unnecessary or undesired trauma to tissue. For example, the proximal anchors tips 26 and distal anchor tips 28 may form flat, substantially flat, curved or other non-sharp surfaces to allow the tips to engage and/or grasp tissue, without necessarily piercing or puncturing through tissue. A looped end or looped anchor may assist the frame in not getting caught up on structures at or near the treatment location. For example, each loop can be configured so that when the frame is deployed in-situ and the anchors expand away from the frame, the movement of each loop from a delivered position to a deployed position avoids getting caught on the papillary muscles.

The prosthesis 10 may include a valve 60 as can be seen in schematically in Figure 6. The valve 60 can be a replacement heart valve which includes a plurality of valve leaflets. The plurality of valve leaflets can function in a manner similar to the natural mitral valve, or to other valves in the vascular system. The plurality of valve leaflets can open in a first position and then engage one another to close the valve in a second position. The plurality of valve leaflets can be made to function as a one way valve such that flow in one direction opens the valve and flow in a second direction opposite the first direction closes the valve. The replacement heart valve 60 can be constructed so as to open naturally with the beating of the heart. For example, the plurality of valve leaflets can open during diastole and close during systole.

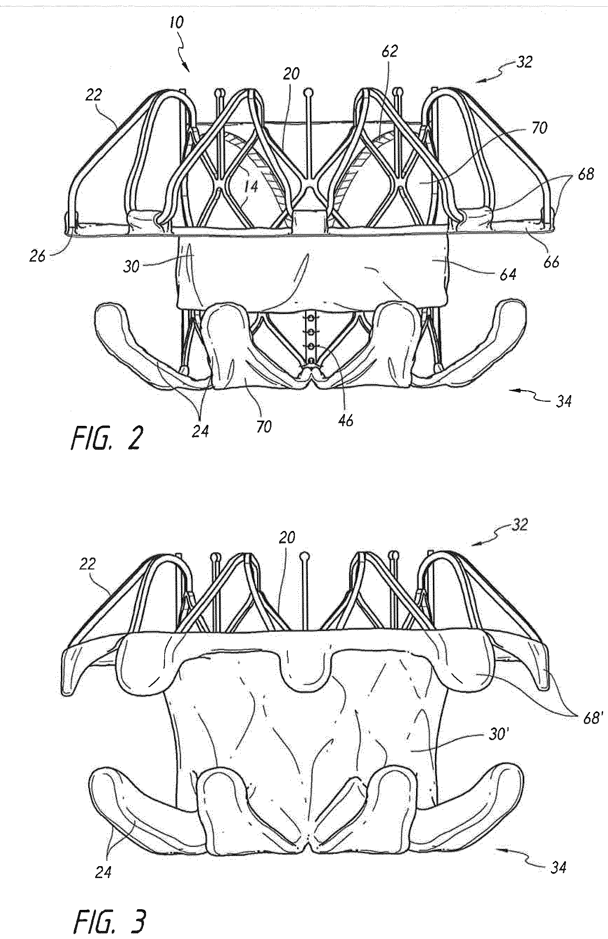

In some embodiments, the leaflets can be coupled to a valve skirt 70. For example, Figure 2 shows a seam 62 where the proximal ends of the leaflets can be connected to the valve skirt 70.

The valve skirt 70 can be used to at least partially control how fluid flows through and/or around the valve 60. The valve skirt 70 can surround at least a portion of the valve and be connected to the valve leaflets. In some embodiments, the valve skirt 70 can form an inner wall connected to and positioned within the frame 20. For example the skirt 70 can connect to the frame at the eyelets 46, such as by stitching. The skirt may also be attached directing to the struts, typically also by stitching. The valve skirt 70 can also be made to move with the foreshortening portion of the frame 20.

The valve skirt 70 can extend the length of the frame 20 or it can extend along only part of the length of the frame 20. In some embodiments, the ends of the heart valve 60 can coincide with ends of the valve skirt 70. In addition, one or more of the ends of the frame 20 can coincide with the ends of the valve skirt 70. In the illustrated embodiment of Figure 2, the proximal end of the valve skirt 70 is positioned proximally from the proximal end of the heart valve 60 as indicated by the seam 62. The valve skirt 70 can not only extend to the distal end of the frame 20 but can also extend to the outside of the frame and is shown attached to and extending to the tip 28 of each distal anchor 24. As shown, the skirt 70 is sewn to each distal anchor.

Other shapes and configurations can also be used for the valve 60 and valve skirt 70. In some embodiments, the valve skirt 70 may extend along the length of the leaflets, but is not connected to them. In the illustrated embodiments, the valve skirt 70 is attached to the frame 20 and the leaflets are attached to the valve skirt 70, such as at the seam 62 in Figure 2.

The valve skirt 70 can be constructed in multiple different ways. The valve skirt 70 can be made a layer of resilient material, such as knit polyester or another stretchable or flexible fabric. In some embodiments, the valve skirt 70 is made from a material that is more flexible than the valve leaflet material. The distal and/or proximal end of the skirt 70 can be straight, curved, or have any other desired configuration. For example, the valve skirt 70 is shown with a straight proximal end at the proximal end 32 of the frame. In other embodiments the skirt distal end can be patterned to generally correspond to the undulations at one end of the frame 20.The valve skirt 70 can be formed of one piece or multiple pieces. For example, the valve skirt 70 attached to the valve 60 can be one piece and then each distal anchor can be covered by a separate piece of material of the valve skirt 70. It is to be understood that other configurations of the valve skirt 70 can also be employed. For example, the anchors may remain uncovered, or only a portion may be covered.

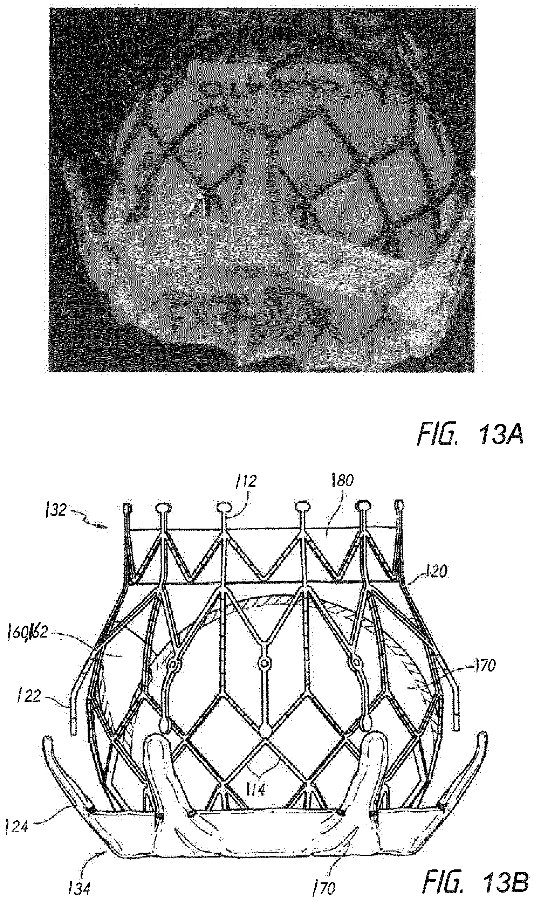

In another embodiment of the valve skirt 70, the end can extend past the frame and can be wrapped around it. Thus, the valve skirt 70 can extend from the inside of the frame 20 to the outside of the frame. The skirt can extend completely around the frame for 1/4, 1/3, 1/2, or more of the length of the distal anchors. Such an embodiment is shown and described with respect to Figures 7A-7B of Provisional Appl. No. 61/782,707, reproduced and described with respect to Figures 13A-13B herein. The skirt 70 can also cover the distal anchors 24 as is shown in Figures 7A and 7B of Provisional Appl. No. 61/782,707 (Figures 13A and 13B herein) and in Figure 2 herein. The skirt can be a one piece skirt, but it will be understood that the skirt can be made of multiple pieces.

The valve skirt 70, and particularly portions that cover the distal anchors 24, can beneficially be used to help prevent leakage of blood flow around the heart valve. In addition, the skirt can encourage tissue in-growth between the skirt and the natural tissue. This may further help to prevent leakage of blood flow around the heart valve.

Continuing to look to Figure 2, an outer skirt or apron 30 is shown that may also form part of the prosthesis 10. Figure 2 shows the outer skirt 30 attached to the frame 20 at the tips 26 of the proximal anchors. The outer skirt 30 can have a portion shaped to correspond generally with the shape of an outer portion of the frame 20. For example, a first portion 64 of the outer skirt 30 can have a cylindrical or generally cylindrical shape with an inner diameter that substantially corresponds in size to, or may be larger or slightly larger than, an outer diameter of the frame 20. The outer skirt 30 can have a second portion 66 with an annular shape that extends away from the first portion 64 to an outer border with a diameter larger than the diameter of the first portion. As illustrated in Figure 2, the second portion 66 is shown flaring outward from the first portion 64 and extending generally perpendicularly from the first portion 64. Thus, the illustrated second portion forms an annular ring comprising a proximal edge and a distal edge, wherein a diameter of the proximal edge is larger than a diameter of the distal edge.

The outer skirt 30 can attach to the frame, and more preferably attach to the anchors, in one of many different ways. The outer skirt 30 can be sewn to the frame and/or valve skirt. The outer skirt 30 can also be wrapped around a portion of the frame and then sewn to itself. In the embodiment illustrated in Figure 2, the second portion 66 is attached to the proximal anchors 22. For example, a plurality of circumferentially spaced tabs 68 extending radially outward from the proximal edge of the second portion 66 can be used to attach the outer skirt 30 to the proximal anchors. The tabs 68 can be wrapped around the tip 26 (e.g., through the loop) of a proximal anchor and connected to the second portion. The tabs 68 themselves may also form sleeves that are configured to surround at least a portion of the proximal anchors. In some embodiments, the proximal anchors 22 can include eyelets that may be used to secure the skirt to the anchor. The tab 68 can be attached to the eyelet 46, for example by stitching.

In one embodiment, the outer skirt 30 is only attached to the frame via the proximal anchors, and the first portion 64 remains unattached to any portion of the frame or any anchors. In another embodiment, the outer skirt is both attached to the proximal anchors and to the middle portion of the frame. As illustrated in Figure 2, the second portion 66 attached to the anchors extends inwardly from the proximal anchors 22. The first portion 64 then extends distally from the second portion 66 and terminates in a distal edge, which may be free or which may attach to the middle portion of the frame 20 or the skirt 70. In other embodiments, the first portion 64 may also be attached to portions of the frame and/or the distal anchors. In some embodiments, the distal edge of the skirt 30 may be spaced radially outward from the frame when the frame is in an expanded configuration. In addition, the distal edge of the skirt 30 may extend to the distal end of the frame, or it may be spaced proximally therefrom as illustrated in Figure 2.

In some embodiments, the outer skirt 30 can attach to the frame at a distal end of the skirt, or at some other location and then curve up and out towards the proximal anchors. Thus, the outer skirt may not have a distinct first portion and second portion. In still other embodiments, the outer skirt may extend along a substantial portion of the frame. Additional examples of outer skirt features that may be incorporated and/or interchanged with the features described herein are found in U.S. Provisional Application No. 61/789,783 filed March 15.

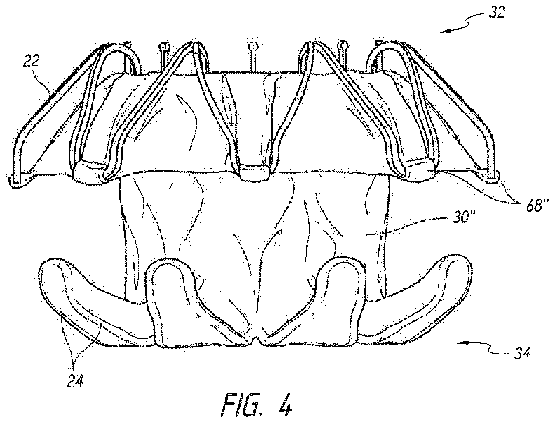

Figures 3 and 4 show additional embodiments of the outer skirt 30', 30". In Figure 3, the outer skirt 30' extends along a substantial part of the frame and extends between proximal and distal anchors. The outer skirt 30' in this embodiment may be one single piece, or may be formed from multiple pieces stitched or otherwise connected together. The tabs 68' may form sleeves that are configured to surround at least a portion of the proximal anchors to attach the outer skirt to the proximal anchors. As shown in one embodiment, proximal portions of the proximal anchors remain uncovered by the outer skirt 30'. The outer skirt 30' at the proximal anchors can form an annular ring similar to the second portion 66 of Figure 2 and can form a substantially cylindrical portion similar to the first portion 64 of Figure 2. The distal end of the outer skirt 30' can attach and/or cover the distal anchors 24. In some embodiments, the distal end of the outer skirt 30' extends to the distal anchors 24 but does not cover or connect to them. In addition, a valve skirt 70 may also be used which may create some overlap of skirts in some embodiments.

In Figure 4 the outer skirt 30" is shown attached to the distal ends of the proximal anchors 22 with tabs 68". The outer skirt 30" then extends proximally to essentially the base of the proximal anchor. From that point, the outer skirt extends distally towards the distal end. Again the outer skirt may or may not attach to the distal anchors. In the illustrated embodiment of Figure 4, the outer skirt 30" wraps around each of the distal anchors.

Turning now to Figure 6, a detail view of an embodiments is shown where the proximal anchors 22 can include eyelets 46 that may be used to secure the skirt to the anchor. In this embodiment, the proximal anchor has an end 26 that instead of extending generally distally, it extends generally radially outwardly, and as illustrated extends in a direction perpendicular or substantially perpendicular to the longitudinal axis of the frame. The tab 68 can be attached to the eyelet 46, for example by stitching. As shown, the eyelet 46 is positioned at the end of the anchor, but it will be understood that it can be spaced proximally from the end. In some embodiments, the proximal anchors can be looped anchors or have a looped end. A small tab can be passed through the looped anchor or looped end and connected to the skirt to form a loop on the skirt. Further, the outer skirt 30 may attach directly to the eyelets 46 without the need for tabs 68.

In some embodiments, the outer skirt 30 can be part of, or connected to, the valve skirt 70, such as being connected to the valve skirt 70 at or near the distal end 34 of the frame.

The outer skirt 30 can be constructed in multiple different ways and may be made of similar material to the valve skirt 70. The outer skirt 30 can be made of a layer of resilient material, such as knit polyester or another stretchable or flexible fabric. In some embodiments, the outer skirt 30 is made from a material that is more flexible than the valve leaflet material. The distal and/or proximal end of the outer skirt 30 can be straight, curved, or have any other desired configuration. The outer skirt 30 can be formed of one piece or multiple pieces. For example, the outer skirt 30 attached to the frame 20 can be one piece and then each proximal anchor 22 can be covered by a separate piece of material of the outer skirt 30. It is to be understood that other configurations of the outer skirt 30 can also be employed. For example, the anchors may remain uncovered, or only a portion may be covered.

The outer skirt 30 can beneficially prevent axial flow of fluid around an exterior of the prosthesis. For example, with the outer skirt 30 be positioned annularly around an exterior of the expandable frame and secured to at least some of the plurality of proximal anchors, the outer skirt creates an axial barrier to fluid flow exterior to the frame when deployed within a body cavity. In addition, the skirt can encourage tissue in-growth between the skirt and the natural tissue. This may further help to prevent leakage of blood flow around the heart valve.

In one embodiment, the outer skirt 30 can be used to help prevent leakage of blood flow around a heart valve, such as a mitral valve, when the prosthesis is placed in a native heart valve. For example, the outer skirt 30 can engage an atrial side of the mitral valve. The proximal anchors can also engage the mitral valve forcing the outer skirt 30 into close contact with the valve to block flow from passing through the mitral valve from outside of the frame.

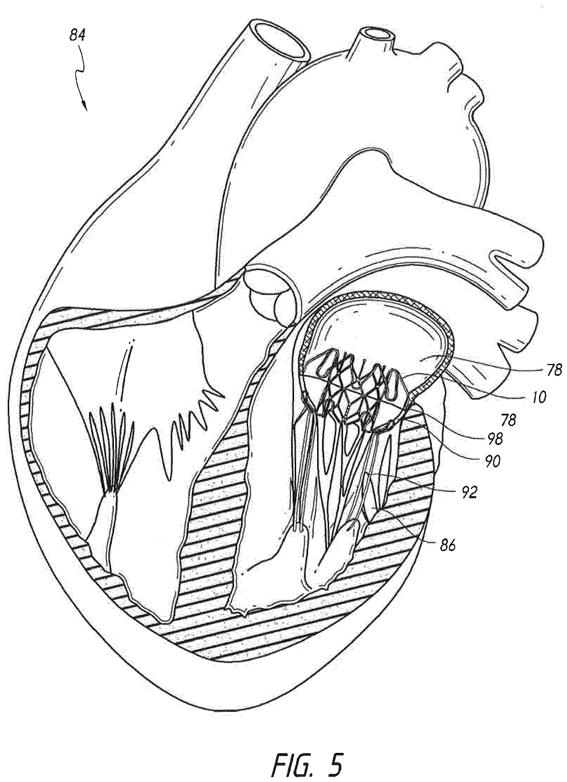

In preferred embodiments, the prostheses 10 in the form of a replacement heart such as described above may be deployed into a heart valve annulus. The prosthesis 10 may be delivered into the mitral valve in a radially compacted or collapsed configuration and positioned when compacted so that the anchor tips 26, 28 of the opposing anchors 22, 24 are disposed on opposite sides of the native annulus 98 as shown in Figures 5 and 6. In Figure 5, the valve 60 and skirts are not shown for ease of illustration. As the replacement heart valve 10 is expanded, the opposing anchors expand outward away from the frame are may be drawn closer together due to foreshortening of the frame. The anchors may grasp tissue on opposite sides of the native annulus 98 and securely hold the replacement heart valve 10 in position. As such, the replacement heart valve 10 can be held securely in position without requiring a substantial radial force against the native annulus. Because the anchor tips are preferably atraumatic, the grasping or engaging of tissue by the prosthesis minimizes damage to the native tissue. The prosthesis can be deployed into a heart valve or otherwise deployed in manners similar to those described with respect to a replacement heart valve in U.S. Publication Nos. 2010/0298931 and 2012/0078353.

Figures 5 and 6 show a schematic representation of the replacement heart valve 10 installed in a human heart 84. The heart is shown in cross-section, and represents typical anatomy, including a left atrium 78 and left ventricle 86. The left atrium 78 and left ventricle 86 communicate with one another through a mitral annulus 98. Also shown schematically is a native anterior mitral leaflet 90 having chordae tendineae 92 that connect a downstream end of the anterior mitral leaflet 90 and to the left ventricle 86. Figure 6 shows an enlarged view of a slightly different prosthesis implanted at the native mitral annulus.

A method of delivering a replacement valve to a native mitral valve and atraumatically securing the replacement valve relative to the native mitral valve annulus 98 is described as follows. The replacement valve can be mounted on a delivery device and delivered to the native mitral valve annulus while the replacement valve is in a radially compacted state. The replacement valve may be positioned so that the ends or tips of the distal anchors are on a ventricular side of the native leaflets 90 beyond a location where chordae tendineae 92 connect to free ends of the native leaflets. At least a portion of the replacement valve can be released from the delivery device to thereby expand the distal anchors radially outwardly. At this time the distal anchors may extend between at least some of the chordae. The distal anchors (along with the frame) can be moved toward the ventricular side of the native valve annulus with the distal anchors extending between at least some of the chordae tendineae to provide tension on the chordae tendineae. With tension provided on the chordae tendineae, the replacement valve can be further released from the delivery device to thereby expand the proximal anchors radially outwardly. The proximal anchors upon further release of the replacement valve from the delivery device can move into engagement with tissue on an atrial side of the native valve annulus, such as with the atrial side of the native valve annulus.

The method just described may utilize any of the prostheses herein described. The illustrated prosthesis where the ends of the distal anchors are not positioned as far out radially as the ends of the proximal anchors when the frame is expanded can beneficially be used in this method. Thus, the distal anchors may have a suitable length for extending between and providing tension on the chordae tendineae, but need not and may in some embodiments not engage tissue with the tips 28, such as shown in Figure 6. Thus, in some embodiments some or all of the distal anchors remain spaced from tissue on the ventricular side of the native valve annulus after delivery and expansion. The interaction between the distal anchors and the chordae tendineae may therefore be sufficient to secure the distal end of the prosthesis, while the engagement of the proximal anchors with tissue on the atrial side of the native valve annulus will help further secure and orient the prosthesis

As illustrated in Figures 5 and 6, the distal anchors may comprise loops, such as any of the looped structures previously described. The proximal and/or distal anchors may also be covered with a resilient material such as described above for the outer skirt 30 and valve skirt 70 that promotes tissue growth with adjacent body tissue. Such material may also be useful to prevent paravalvular leakage. The atraumatic distal anchors may advantageously prevent snagging of the prosthesis on internal structures, such as the papillary muscles.

When the prosthesis is in an expanded configuration within the native mitral heart valve, the engagement of the proximal anchors 22 with tissue on the atrial side of the native mitral valve causes at least a portion of the outer skirt 30 to also engage the tissue on the atrial side of the native mitral valve. A portion of the outer skirt extends distally from the proximal anchors toward the ventricle. Because the diameter of the outer skirt decreases to a size of close or the same in dimension as the frame, the outer skirt form a barrier to blood flow around the outside or external to the frame. The outer skirt 30 can be forced against the outside of the frame 20 by the native leaflets. Where the native leaflets do not force the outer skirt 30 against the frame, or where the contact is not as strong, the outer skirt 30 is still present to block, or impede blood flow. It will be understood that having multiple contact points between the native valve and the outer skirt can allow the outer skirt to securely cover areas where there are fewer contacts between the two. As described above, the outer skirt may also promote tissue growth with tissue that it contacts.

In addition, due to the preferred outer dimension of the anchors relative to the diameter or radius of the frame in some embodiments, when the frame is radially expanded such that the proximal and/or distal anchors engage tissue at or around the native mitral valve annulus, the frame may move reciprocally in an axial direction relative to the native mitral valve annulus in a constrained floating manner. The one or more of the anchors 22, 24 can be made to flex to provide this reciprocal movement; for example, around the bends between the segments 50, 56 and the base of the anchors. The frame does not exert a significant amount of radial force to the native mitral valve annulus or adjacent tissues, and the frame is primarily secured with the anchors. When in use, the frame may then move relative to the anchor ends as the heart is beating.

The embodiments described in Figures 1-6 above may have further advantages. As illustrated in Figure 1, some embodiments have only 3 rows of cells or less, which makes the prosthesis longitudinally shorter and therefore easier to navigate when collapsed through tortuous pathways (e.g., percutaneously). Because of the dimensions of the anchors relative to the size of the frame, the frame itself may be made relatively smaller, which also helps facilitate a lower profile for the prosthesis helpful for delivery and implantation. Moreover, having a prosthesis that can "float" within a native annulus may be usable for a wider variety of patient anatomies, as one or a fewer number of radial sizes of the frames can be used to fit a greater number of patients. In such embodiments, because the anchors are configured to extend further from the frame, these prostheses are still able to securely grasp native tissue as the anchors can expand to different diameters depending on how they are constrained with a body cavity. In the context of a replacement heart valve, the frame (and the associated valve body) may have the same size across multiple patient sizes, and the anchors can either be configured to expand to different diameters, or different anchor arrangements may be used for different frames.

With reference to Figure 7A, an embodiment of a prosthesis 110 is shown. The illustrated prosthesis 110 includes a frame 120 that may be self-expanding or balloon expandable. The prosthesis may further include a replacement valve that can be designed to replace a damaged or diseased native heart valve such as a mitral valve. The replacement valve is not shown in this embodiment as to more clearly illustrate features of the frame 120, though it will be understood that a replacement valve is not required as part of the prosthesis. In addition, it will be understood that only a front portion of the frame 120 is shown for further ease of illustration.

The frame 120 can be made of many different materials, but is preferably made from metal. In some embodiments, the frame 120 can be made from a shape memory material, such as nitinol. A wire frame or a metal tube can be used to make the frame. The wire frame of a metal tube can be cut or etched to remove all but the desired metal skeleton. In some embodiments a metal tube is laser cut in a repeating pattern to form the frame. Figure 7B illustrates the flat cut pattern of the frame shown in Figure 7A. The flat pattern can be cut from a metal tube and then the tube can be bent and expanded to the shape shown in Figure 7A. The frame 120 can further be expanded and/or compressed and/or otherwise worked to have the desired shape or shapes, such as for introduction and implantation.

As shown, the frame when in an expanded configuration, such as in a fully expanded configuration, has a bulbous or slightly bulbous shape, with a middle portion being larger than the proximal 132 and distal 134 ends. In some embodiments, the inside diameter of the both ends can be the same, or it can be bigger on one end than the other, while still having a middle portion larger than both the proximal and distal ends. In some embodiments, the effective diameter of the distal frame end is smaller than the effective diameter of the middle portion. The bulbous shape of the frame can advantageously allow the frame to engage a native valve annulus or other body cavity, while spacing the inlet and outlet from the heart or vessel wall. This can help reduce undesired contact between the prosthesis and the heart or vessel, such as the ventricular wall of the heart. In other embodiments, the frame may not have a bulbous portion, and can have substantially the same outer dimension along its entire length, or it may have one end larger than the other end. The prosthesis 110 and frame 120 may be similar to the replacement heart valves and associated frames disclosed in U.S. Patent No. 8,403,983 and U.S. Publication Nos. 2010/0298931, 2011/0313515 and 2012/0078353.

A number of struts collectively make up the frame 120. Figure 7A illustrates the frame in an expanded configuration with a number of longitudinal struts 112 and undulating struts 114, with cells defined by the open spaces between the struts. The longitudinal struts may be arranged so that they are parallel or generally or substantially parallel to a longitudinal axis of the frame. The longitudinal axis of the frame may be defined as the central axis that extends through the center of the frame between the proximal 132 and distal 134 ends. Any number of configurations of struts can be used, such as the rings of undulating struts shown forming chevrons and diamonds, but also ovals, curves, and various other shapes. The illustrated embodiment includes two rings, or rows of chevrons shown in portion 116 and two rows of diamond-shaped cells shown in portion 118.

The frame 120 has a non-foreshortening portion 116 and a foreshortening portion 118. These portions can be defined by the frame 120 and the positioning of various types of struts along the frame 120. In Figure 7A it can be seen that the longitudinal struts 112 span the length of the non-foreshortening portion 116, while undulating struts 114 form the foreshortening portion 118. When the frame is radially collapsed or compacted, the struts 114 become more parallel with respect to the longitudinal axis of the frame, causing an outer diameter of the frame to decrease and the longitudinal length of the frame to increase in the foreshortening portion 118. As the frame moves from a compacted position to an expanded position, the longitudinal length of the frame can decrease in the foreshortening portion 118. But, the frame length does not substantially change length in the non-foreshortening portion 116.

Foreshortening of the frame 120 can be used to engage and secure the prosthesis to intralumenal tissue in a body cavity, for example tissue at or adjacent a native valve, such as a native valve annulus and/or leaflets. Opposing anchors 122, 124 can be constructed on the frame 120 so that portions of the anchors, such as tips or ends 126, 128, move closer together as the frame foreshortens. As one example, this can allow the anchors 122, 124 to grasp tissue on opposite sides of the native mitral annulus to thereby secure the prosthesis at the mitral valve.

The anchors 122, 124 and anchor tips 126, 128 can be located anywhere along the frame 120 just so long as at least one of the anchors is either connected to the foreshortening portion 118 or the foreshortening portion is positioned between the anchors so that a portion of the anchors will be move closer together with expansion of the frame. As shown, the anchors 124 are connected to the foreshortening portion 118. The foreshortening portion can also be positioned anywhere along the frame, though it is shown towards the distal end 134. In some embodiments, both of the anchor tips 126, 128 are located in the foreshortening portion 118. In some embodiments, the foreshortening portion 118 may extend the entire length of the frame, such that there is no non-foreshortening portion 116.

Preferably, each of the anchors 122, 124 is positioned or extends generally radially outwardly from the frame 120 so that the anchor tips 126, 128 are generally spaced away or radially outward from the rest of the frame 120. For example, the anchor tips may be located radially outward from the middle portion of the frame, with the tips 126 and 128 being axially spaced from one another. In some embodiments, all or part of the structure connected to the anchor tip and extending radially from the frame, including one or more rings and/or struts, can be considered part of the anchor. The anchors can include a base located on the anchor on a side opposite the tip. The base can be for example where the anchor begins to extend from or away from the frame 120.

For example, proximal anchors 122 are shown having first 136 and second 38 struts forming a chevron and connected to longitudinal struts 112 at a base of the anchor. The first and second struts of the anchor 122 are bent at the base so that the anchor 122 extends radially outwardly from the frame as it extends generally distally towards the tip 126. The first and second struts can be connected to each other at a radially outward location to form an outwardly extending loop, and in some embodiments, the first and second struts can be joined at a third strut 140 that continues to extend outwardly and/or generally distally. Here the third strut 140 is a short strut. The anchor also includes an eyelet 146. As illustrated, the eyelet is located at the distal end 126, though the eyelet can be positioned in other locations along the anchor 122. The tips 126 of the proximal anchors may extend distally and be parallel or substantially parallel with the longitudinal axis of the frame, or as illustrated in Figure 7A, the tips 126 may extend generally distally but still radially outwardly inclined or at an acute angle relative to the longitudinal axis of the frame.

As another example, the distal anchors 124 are shown having looped ends 148. The looped ends can be larger near the tip to form a type of elongated teardrop. In addition, the tips 128 may be substantially flat. The looped end may assist the frame in not getting caught up on structures at or near the treatment location. For example, each loop can be configured so that when the frame is deployed in-situ and expands, the movement of each loop from a delivered position to a deployed position can avoids getting caught on the papillary muscles.

Each distal anchor 124 is connected to the frame at a base 142. As illustrated in Figure 7A, the base of the distal anchor may be at a location where the corners of adjacent cells meet, such that the base is proximal to the distal end 134 of the frame. In other embodiments, the base of the distal anchor may be at a distal most corner of a cell, which corresponds to a distal most point on the frame The distal anchors as illustrated extend from the base 142 generally distally before bending back around in an arcuate segment where the distal anchor extends generally proximally and radially outwardly from the frame. As shown, the anchors 124 may also generally distally and radially inwardly with respect to the frame such that the distal most point on the prosthesis has a smaller inside diameter than where the base 142 connects to the frame. The inside diameter at the distal most can be the same or substantially the same as the inside diameter of the proximal end, or may be smaller. The anchor as illustrated is bent around about 180 degrees so that the tip 128 extends in the opposite, proximal direction, which may be parallel or substantially parallel to the longitudinal axis of the frame. For example, in Figure 7A it can be seen that the distal anchors 124 are bent further inward such that the ends of the anchors point proximally and are generally parallel with the longitudinal axis of the frame. Alternatively, the tip 128 may extend generally proximally but still extend radially outwardly inclined or at an acute angle relative to the longitudinal axis of the frame

It will be understood that the anchors can have various other configurations, including the various embodiments that follow. In some embodiments, each of the anchors can extend radially outwardly from the frame at an anchor base and terminate at an anchor tip. The anchors can be connected to the frame at one of many different locations including apices, junctions, other parts of struts, etc. The anchors can comprise first, second, third, or more spaced apart bending stages along the length of each anchor. The anchors can also extend either distally or proximally before and/or after one or more of the bending stages. A portion of the anchor may extend with the frame before or after any bending stages.

In the illustrated embodiment of Figure 7A-B there are twelve distal anchors and twelve proximal anchors. In some embodiments there may be 116 anchors on one side and 112 on the other. Some embodiments may include different numbers of anchors. In addition, the distal and proximal anchors may be aligned so the tips point generally towards each other, or they may be spaced so that the tips point between two tips on the opposite side, as is illustrated in Figures 7A-B.

The anchor tips 126 and 128 as described above advantageously provide atraumatic surfaces that may be used to grasp intralumenal tissue without causing unnecessary or undesired trauma to tissue. For example, the proximal anchors tips 126 and distal anchor tips 128 may form flat, substantially flat, curved or other non-sharp surfaces to allow the tips to engage and/or grasp tissue, without necessarily piercing or puncturing through tissue.

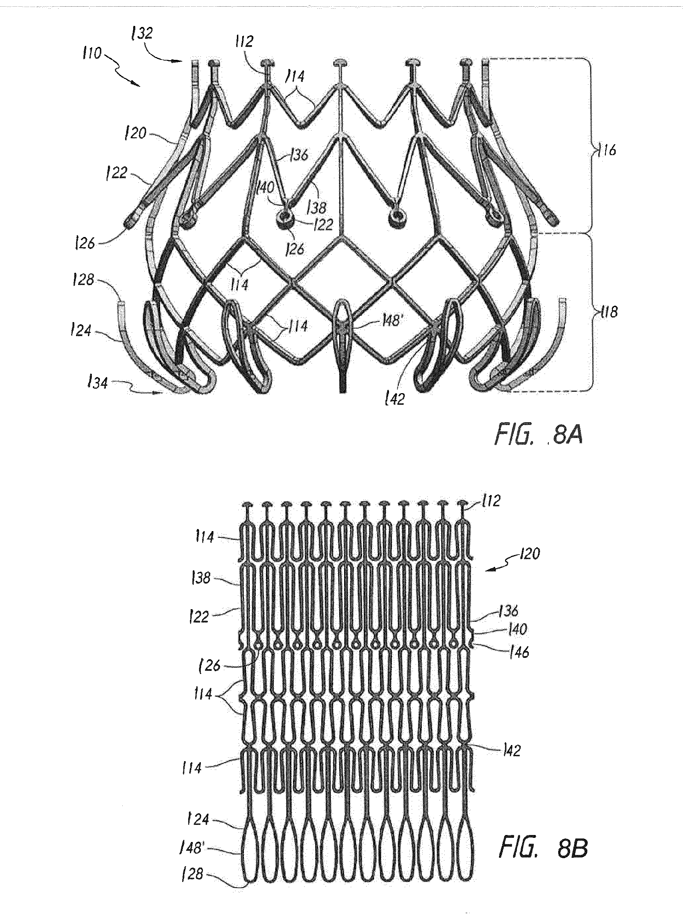

Figures 8A-9B show prostheses similar to that of Figures 7A-B with two different styles of distal anchors 124. In Figures 8A-B, the looped end 48' of the distal anchor is generally more elliptical with a curved tip as compared to the elongated teardrop shape of looped end 148 of Figures 7A-B. Otherwise the shape is substantially the same.

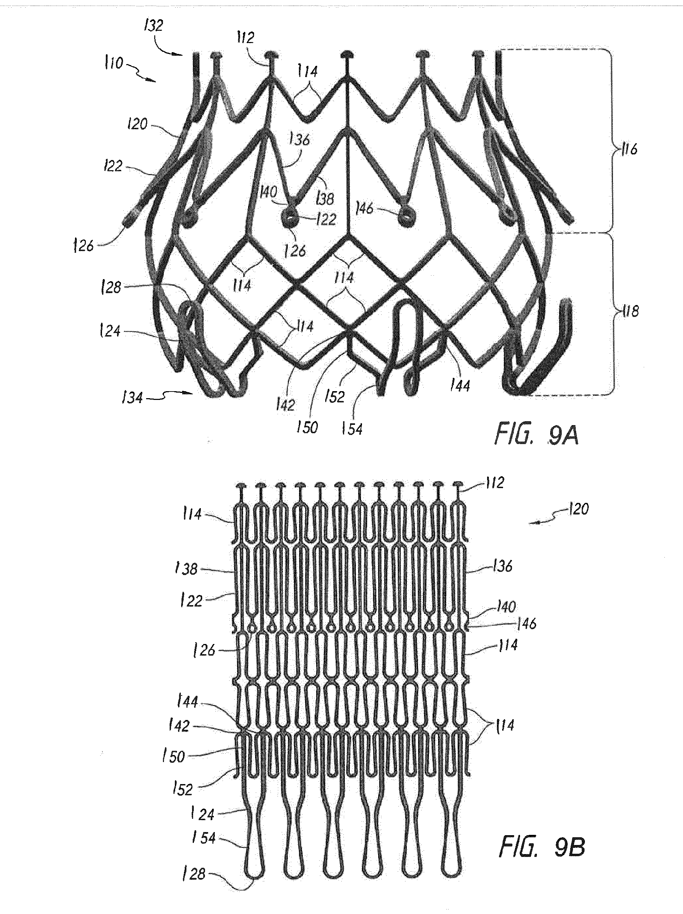

In Figures 9A-B, the distal anchors 124 are looped anchors rather than having looped ends. The looped anchor has a first base 142 and a second base 144 connected to the frame, wherein the first and second bases are at opposite corners of the same cell. Alternatively, the first and second bases may be located at the distal most corners of adjacent cells. The distal anchors 124 extends generally distally from the frame at the first base 142 but then is bent back around and begins to extend outwardly from the frame in a generally proximal direction. The distal anchor 124 then repeats this configuration in reverse towards the second base 144 such that the two sides of the looped anchor are mirror images of one another. It will be understood that the looped anchor can have other configurations and that it may not be symmetrical.

As illustrated in Figure 9A, the tips 128 of the distal anchors are circumferentially aligned with the tips 126 of the proximal anchors, though in other embodiments, the tips 128 of the distal anchors may be circumferentially staggered between the tips 126 of the proximal anchors. In the embodiment of Figure 9A, adjacent distal anchors 126 are spaced apart by one cell, though in other embodiments, adjacent distal anchors may be provided on adjacent cells. Thus, for example, instead of having six distal anchors and twelve proximal anchors as shown in Figure 3A, there may be a 1:1 correspondence between proximal and distal anchors.

The illustrated looped distal anchor of Figures 9A-B is made up of the following segments. The first segment 150 extends generally longitudinally with the frame, extending distally or generally distally (e.g., slightly radially inward) with the frame. The strut is then bent so that a second segment 152 extends generally parallel with an adjacent undulating strut 114. The strut is then bent so that a third segment 154 begins to extend generally longitudinally and distally or generally distally, and then is bent back around to point in generally the opposite direction (e.g., in a proximal direction parallel or generally parallel with the longitudinal axis of the frame). The third segment 154 ends in the rounded tip 128 and then the anchor strut repeats to form the mirror image. After the third segment 154 bends back around to point in generally the opposite direction, in the embodiment illustrated the third segment may first extend radially outward at an acute angle relative to the longitudinal axis before bending into a portion that extends parallel or substantially parallel to the longitudinal axis. The paired third segments 154 extend parallel or generally parallel with one another from the second segment to the tip, though they may also move slightly towards or away from each other in some embodiments.

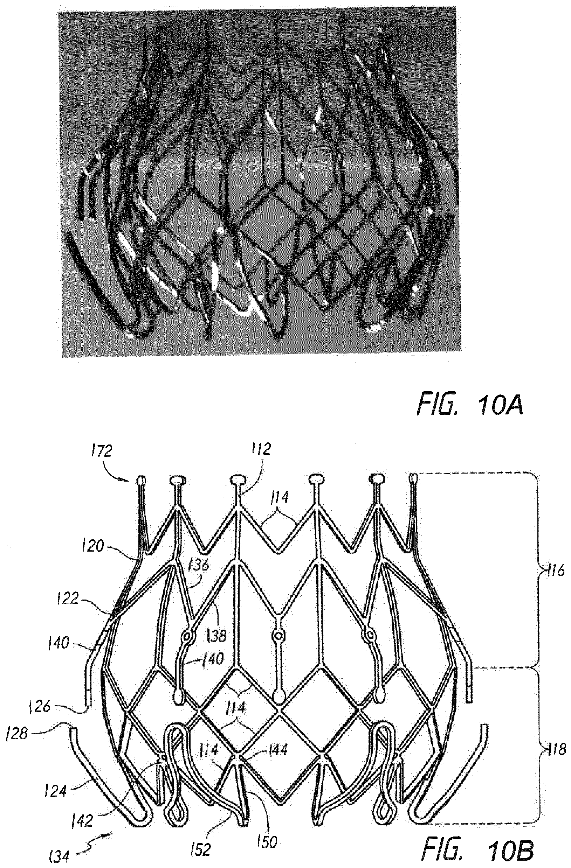

Figures 10A-B show a prosthesis similar to Figures 9A-B that also has looped distal anchors. In this embodiment the first segment 150 extends longitudinally in a distal direction from the frame and the strut is bent back on itself to point generally in the opposite (e.g., proximal) direction. The second segment is bent inward before extending parallel or generally parallel with its mirror image on the other side forming a nose and wing configuration similar to the shape of certain bicycle seats.

The proximal anchors 122 also have an elongated third strut 140. The proximal anchor 122 is shown having first 136 and second 138 struts forming a chevron and connected to longitudinal struts 112 at a base of the anchor. The first and second struts of the anchor 122 are bent at the base so that the anchor 122 extends radially outwardly from the frame as it extends towards the tip 126. The first and second struts join at a third strut 140 that continues to extend outwardly and is then bent such that the tip points distally and extends in a manner parallel or generally parallel with the longitudinal axis of the frame. The proximal anchor may or may not include an eyelet 146 along its length. The distal tip of the proximal anchors may have an atraumatic surface, such as an enlarged circular or curved end as illustrated. When the frame is in an expanded configuration, the distal anchors 124 may have tips1 128 that are positioned radially outward of the tips 126 of the proximal anchors 122. Other embodiments may have the tips 126 being positioned outward of the tips 128. Such configurations are also possible with the other frames and prostheses described elsewhere herein.

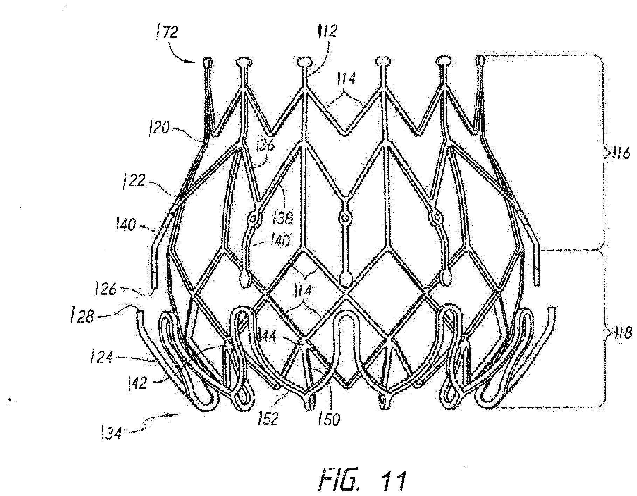

Figure 11 illustrated an embodiment similar to the prosthesis of Figure 10B with twelve distal anchors instead of six. Because of this change, in one embodiment two anchors share the first segment 150 where the anchor base 142, 144 is connected to the frame. As illustrated, each of the proximal and distal anchors may be circumferentially aligned with each other, and each of the distal anchors corresponds to one of the cells

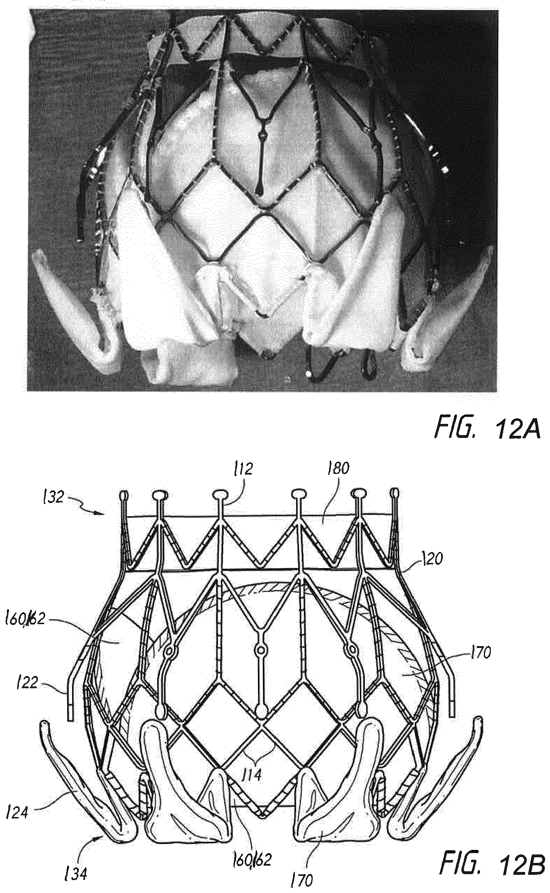

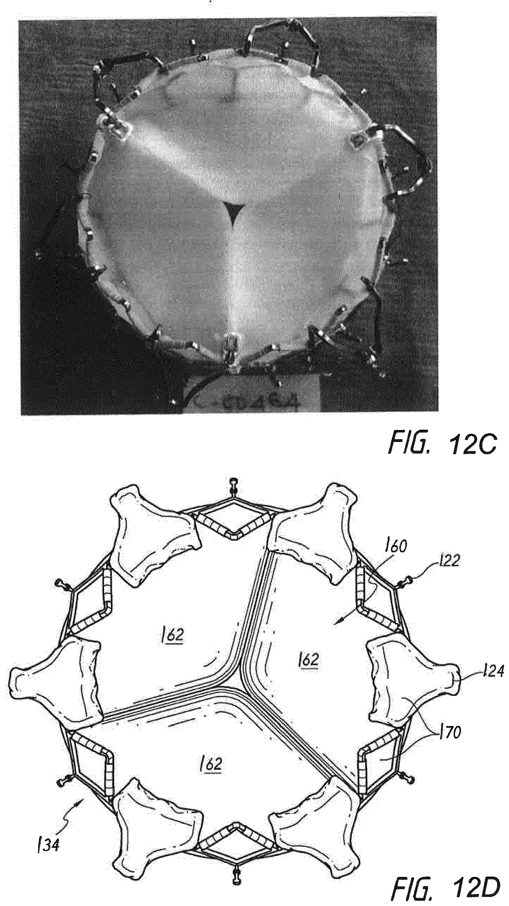

Turning now to Figures 12A-D, prosthesis and frame embodiments are shown similar to that of Figures 10A-B, including various other components of the prosthesis. A prosthesis can include one or more of a valve1 60, a skirt 170 and a support band 180. The prosthesis can be a replacement heart valve similar to that and including features similar to those disclosed in U.S. Patent Appl. Nos. 13/165,721, filed June 21, 2011, published as U.S. 2011/0313515; and 13/244,080, filed September 23, 2011, published as 2012/0078353.

The valve 160 can be a replacement heart valve which includes a plurality of valve leaflets 162. The plurality of valve leaflets 162 can function in a manner similar to the natural mitral valve, or to other valves in the vascular system. The plurality of valve leaflets 162 can open in a first position and then engage one another to close the valve in a second position. The plurality of valve leaflets 162 can be made to function as a one way valve such that flow in one direction opens the valve and flow in a second direction opposite the first direction closes the valve. The replacement heart valve 160 can be constructed so as to open naturally with the beating of the heart. For example, the plurality of valve leaflets 162 can open during diastole and close during systole.

In some embodiments, the leaflets 162 can be coupled to a skirt 170. For example, the proximal ends of the leaflets 162 can be connected to a proximal end of the skirt 170.

The skirt 170 can be used to at least partially control how fluid flows through and/or around the valve 160. The skirt 170 can surround at least a portion of the valve and be connected to the valve leaflets 162. In some embodiments, the skirt 170 can form an inner wall connected to and positioned within the frame 120. The skirt 170 can also be made to move with the foreshortening portion 118 of the frame 120.

The skirt 170 can extend the length of the frame 120 or it can extend along only part of the length of the frame 120. In some embodiments, the ends of the heart valve 160 can coincide with ends of the skirt 170. In addition, one or more of the ends of the frame 120 can coincide with the ends of the skirt 170. In the illustrated embodiment of Figures 12A-D, the proximal end of the skirt 170 and heart valve 160 are sewn together. The skirt 170 can not only extend to the distal end of the frame 20 but can also extend to the outside of the frame and is shown wrapped around each of the distal anchors 124.

Other shapes and configurations can also be used for the valve 160 and skirt 170. In some embodiments, the skirt 170 may extend along the length of the leaflets 162, but is not connected to them. In the illustrated embodiments, the skirt 170 is attached to the frame 120 and the leaflets 162 are attached to the skirt 170.

The skirt 170 can be constructed in multiple different ways. The skirt 170 can be made of knit polyester or another stretchable or flexible fabric. In some embodiments, the skirt 170 is made from a material that is more flexible than the valve leaflet material. The distal and/or proximal end of the skirt 170 can be straight, curved, or have any other desired configuration. For example, the skirt 170 is shown with undulations patterned to generally correspond to the undulations at the distal end 134 of the frame 120. It can be seen that the skirt 170 wraps around the struts at the distal end. The skirt 170 can be formed of one piece or multiple pieces. For example, the skirt 170 attached to the valve 160 can be one piece and then each distal anchor can be covered by a separate piece of material of the skirt 170. This is illustrated in Figure 12A as one of the distal anchors on the back side remains uncovered. In addition, Figure 12C shows the distal anchors uncovered. It is to be understood that other configurations of the skirt 170 can also be employed. For example, the anchors may remain uncovered, or only a portion may be covered.

Turning now to Figures 13A-B, another embodiment of the skirt 170 is shown. Here rather than the skirt 170 corresponding to the undulations at the distal end 134 of the frame 120, the skirt extends past the frame and is then wrapped around it. Thus, the skirt 170 extends from the inside of the frame 120 to the outside of the frame. The skirt can extend completely around the frame for 1/4, 1/3, 1/2, or more of the length of the distal anchors. The skirt can also cover the distal anchors 124. In the illustrated embodiment, the skirt is a one piece skirt, but it will be understood that the skirt can be made of multiple pieces.

The skirt 170, and particularly portions that cover the distal anchors 124, can beneficially be used to help prevent leakage of blood flow around the heart valve. In addition, the skirt can encourage tissue in-growth between the skirt and the natural tissue. This may further help to prevent leakage of blood flow around the heart valve.

The prosthesis 110 can also include a support band 180 as is shown in Figures 12A-13B. The support band 180 may be placed or positioned around or within the frame 120 at the proximal end 132. The support band 180 can be used to reinforce and/or constrain the frame 120. The support band 180 can help to control the expansion of the frame 120 from the compacted to the expanded state. The support band 180 can also be used to reduce the amount of motion that occurs at the proximal end 132 after the prosthesis 110 has been implanted at the mitral heart valve or other location.

In some embodiments, the support band 80 may comprise a polyester fabric band. The support band 180 may comprise a no-stretch or limited stretch material. Preferably the support band 180 is not made of an elastic material or a material known to have high elasticity. In some embodiments, the support band 180 is made from a material that is less flexible than the valve skirt material and/or the valve leaflet material. The distal and proximal ends of the support band 180 can be straight, curved, undulating with the undulations of frame, or any other desired configuration.

The support band 180 can be connected to the valve frame with a plurality of stitches, loops, knots, staples, or other types of connections. In some embodiments, the frame 120 can be sandwiched between two sides or layers of the support band 180. Preferably, the support band 180 is a single layer positioned within and attached to the frame 120 with a plurality of stitches around one or more of the longitudinal and/or undulating struts. In some embodiments, the support band 80 can be attached to the proximal end of the valve skirt 140.

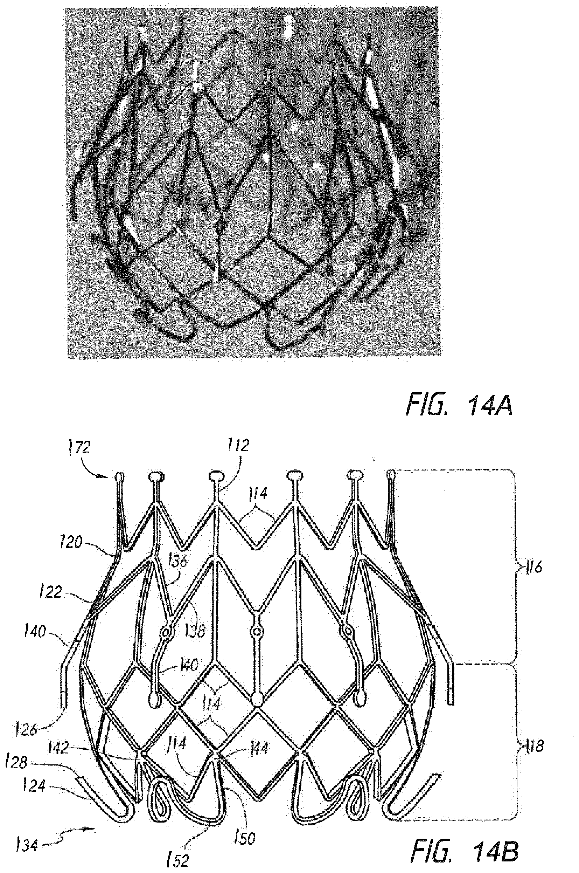

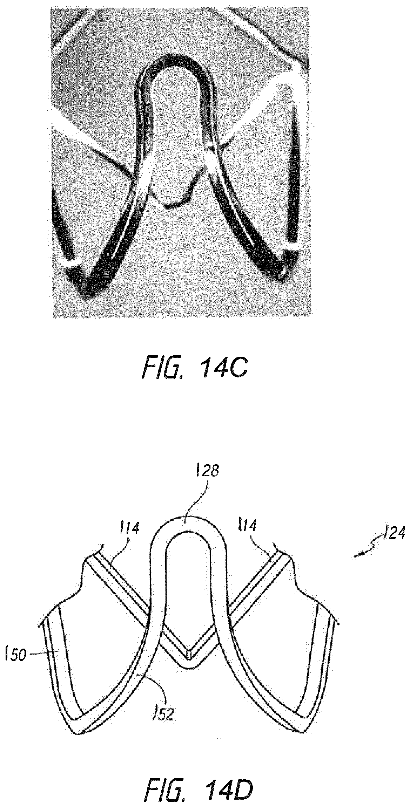

Looking now at Figures 14A-D another embodiment of a prosthesis 110 is shown. Figures 14A-D show a prosthesis similar to that of Figures 10A-B with a different style and configuration of distal anchor 124. In Figures 14A-D, the distal anchors are shorter than and spaced radially inward from the distal anchors of Figures 10A-B. Thus, as illustrated, the distal anchors 124 are not positioned as far radially outward as the proximal anchors, and the tips 128 may be positioned radially inward of the tips 126. As described further below, such a configuration may be advantageous in positioning and securing the prosthesis in a mitral valve or other body location. As shown particularly in Figures 14C and 14D, the distal anchors 124 may comprise loops as described above, having a curved or arcuate atraumatic tip to minimize damage to body tissue.

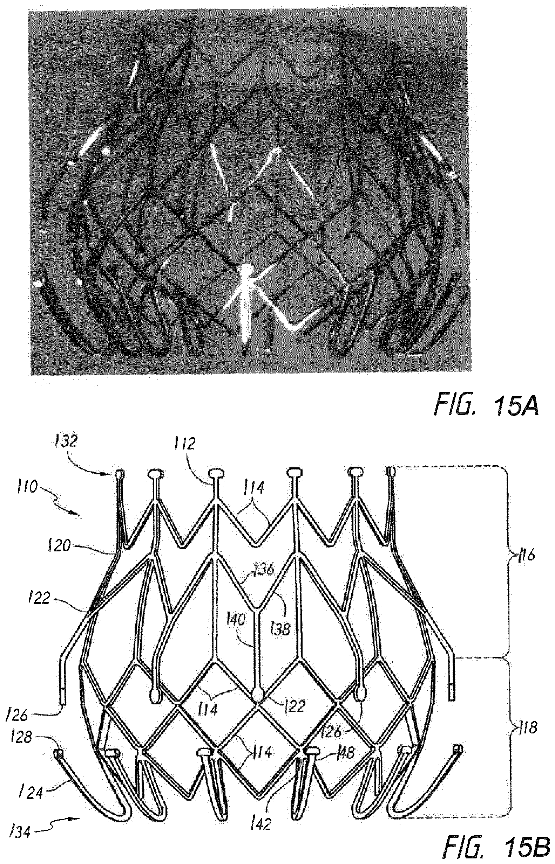

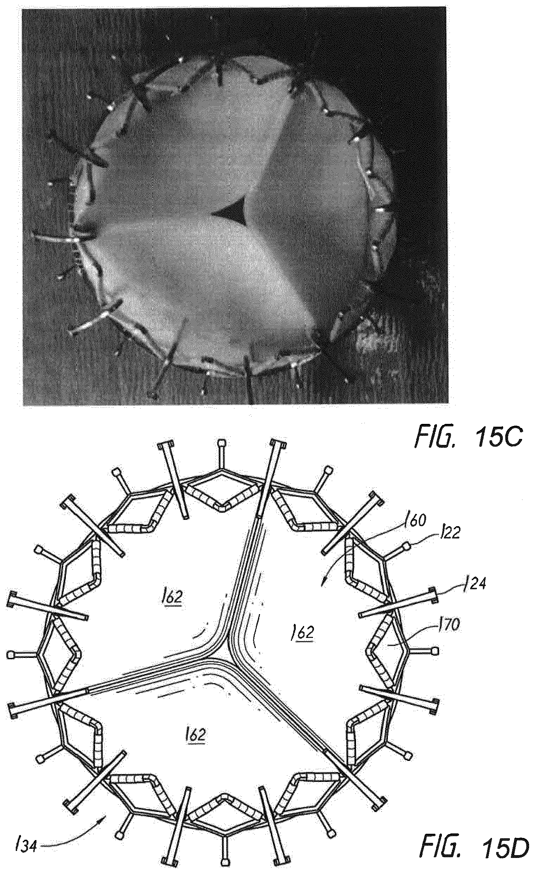

Figures 15A-D show an embodiment of a prosthesis where the distal anchors do not comprise loops, but instead comprise single struts each extending distally from the corners where adjacent cells meet. As described with respect to embodiments above, these anchors may first extend distally or generally distally, and may further extend radially inward, before bending around to extend proximally or generally proximally, such as at an acute angle relative to the longitudinal axis of the frame. The tips 128 of the anchors may comprise an atraumatic surface, such as a flattened or curved enlarged tip. As illustrated, the tips 128 may be circumferentially staggered between tips1 126 of the proximal anchors 122, as best shown in Figures 15C and 15D. Figures 15C-D also show the frame 120 having a valve 160 and skirt 170 attached as described above.

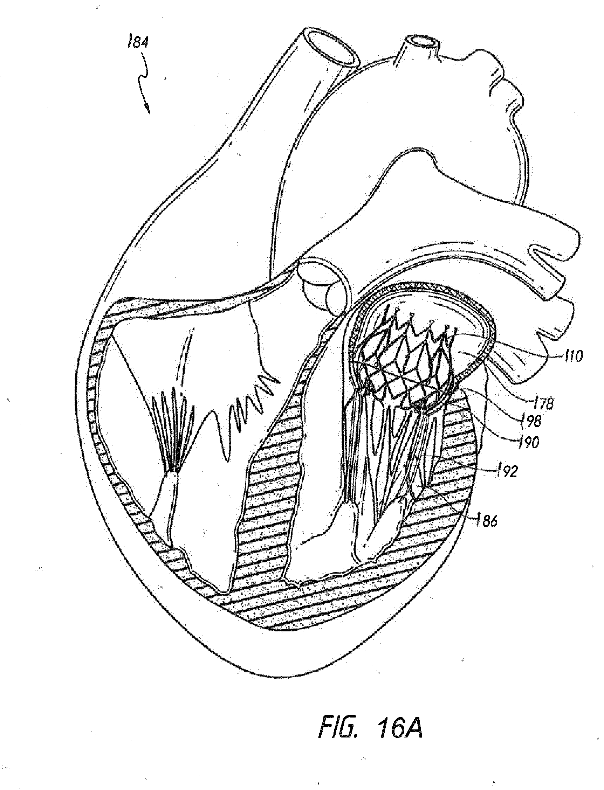

In preferred embodiments, any of the prostheses 110 described above may be deployed into a heart valve annulus, and positioned when compacted so that the anchor tips 126, 128 of the opposing anchors 122, 124 are disposed on opposite sides of the native annulus 188 as shown in Figures 16A and 16BA. As the replacement heart valve 110 is expanded, the opposing anchors are drawn closer together so as to grasp tissue on opposite sides of the native annulus 188 and securely hold the replacement heart valve 110 in position. As such, the replacement heart valve 110 can be held securely in position without requiring a substantial radial force against the native annulus. Because the anchor tips are preferably atraumatic, the grasping or engaging of tissue by the prosthesis minimizes damage to the native tissue. The foreshortening portion 118 can be used to move the anchor tips 126, 128 closer together as the replacement heart valve 110 moves to the expanded position to thereby engage the native valve annulus. The prosthesis can be deployed into a heart valve or otherwise deployed in manners similar to those described with respect to a replacement heart valve in U.S. Publication No. 2010/0298931 and 2012/0078353.

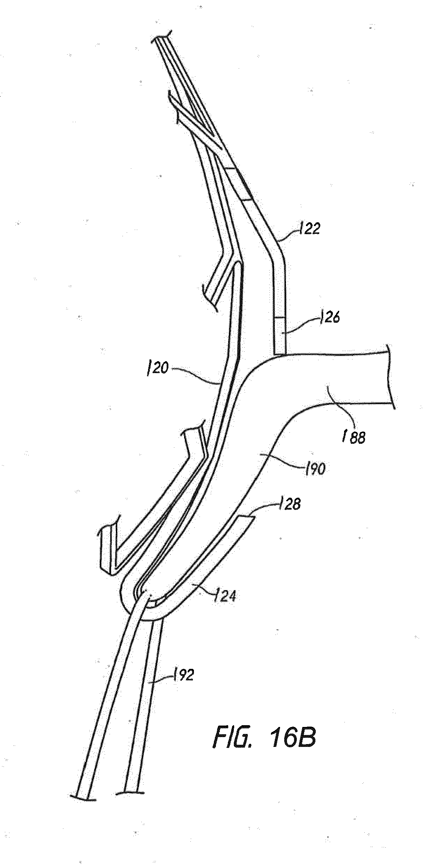

Figures 16A and 16B show a schematic representation of the replacement heart valve 110 installed in a human heart 184. The heart is shown in cross-section, and represents typical anatomy, including a left atrium 178 and left ventricle 186. The left atrium 178 and left ventricle 186 communicate with one another through a mitral annulus 188. Also shown schematically is a native anterior mitral leaflet 190 having chordae tendineae 192 that connect a downstream end of the anterior mitral leaflet 190 and to the left ventricle 186.

A method of delivering a replacement valve to a native mitral valve and atraumatically securing the replacement valve relative to the native mitral valve annulus 188 is described as follows. The replacement valve can be mounted on a delivery device and delivered to the native mitral valve annulus while the replacement valve is in a radially compacted state. The replacement valve may be positioned so that the ends or tips of the distal anchors are on a ventricular side of the native leaflets 190 beyond a location where chordae tendineae 192 connect to free ends of the native leaflets. At least a portion of the replacement valve can be released from the delivery device to thereby expand the distal anchors radially outwardly. At this time the distal anchors may extend between at least some of the chordae. The distal anchors (along with the frame) can be moved toward the ventricular side of the native valve annulus with the distal anchors extending between at least some of the chordae tendineae to provide tension on the chordae tendineae. With tension provided on the chordae tendineae, the replacement valve can be further released from the delivery device to thereby expand the proximal anchors radially outwardly. The proximal anchors upon further release of the replacement valve from the delivery device can move into engagement with tissue on an atrial side of the native valve annulus, such as with the atrial side of the native valve annulus.

The method just described may utilize any of the prostheses herein described, but may be particularly suitable for the prosthesis of Figures 14A-14D where the ends of the distal anchors are not positioned as far out radially as the ends of the proximal anchors when the frame is expanded. Thus, the distal anchors may have a suitable length for extending between and providing tension on the chordae tendineae, but need not and may in some embodiments not engage tissue with the tips 128. Thus, in some embodiments the some or all of the distal anchors remain spaced from tissue on the ventricular side of the native valve annulus after delivery and expansion. The interaction between the distal anchors and the chordae tendineae may therefore be sufficient to secure the distal end of the prosthesis, while the engagement of the proximal anchors with tissue on the atrial side of the native valve annulus will help further secure and orient the prosthesis

As illustrated in Figures 16A and 16B, the distal anchors may comprise loops, such as any of the looped structures previously described. The distal anchors may also be covered with a resilient material such as described above for the skirt 170 that promotes tissue growth with adjacent body tissue. Such material may also be useful to prevent paravalvular leakage. The atraumatic distal anchors may advantageously prevent snagging of the prosthesis on internal structures, such as the papillary muscles.

## Claims

1. A prosthesis configured to grasp intralumenal tissue when deployed within a body cavity, the prosthesis comprising:
an expandable frame (20) comprising a proximal end (32) and a distal end (34) and a longitudinal axis extending therethrough, the frame (20) configured to collapse radially for delivery and to expand radially upon deployment, wherein when the frame (20) is in an expanded configuration, the frame (20) has a larger cross-sectional dimension in a middle portion of the frame (20) and a smaller cross-sectional dimension in a proximal portion and a distal portion of the frame (20), wherein the middle portion is between the proximal and distal portions;
a plurality of proximal anchors (22) extending from the frame (20) and expandable from a collapsed configuration to an expanded configuration, wherein in the expanded configuration, each proximal anchor (22) extends proximally away from the proximal end (32) of the frame (20) and then turns to extend distally, each proximal anchor (22) extending to an end positioned radially outward from the frame (20); and
a plurality of distal anchors (24) extending from the frame (20) and expandable from a collapsed configuration to an expanded configuration, wherein in the expanded configuration, each distal anchor (24) extends distally away from the distal end (34) of the frame (20) and then turns to extend proximally, each distal anchor (24) extending to an end positioned radially outward from the frame (20);
wherein in the expanded configuration, the ends of each of the plurality of proximal anchors (22) are substantially perpendicular to the longitudinal axis of the frame (20);
wherein in the expanded configuration, each of the plurality of distal anchors (24) is bent near a base of the distal anchor (24) to extend radially outwardly away from the frame (20) along a first segment (50) and is then bent to point generally proximally along a second segment (52) ending in a tip (28).

2. The prosthesis of Claim 1, wherein at least some of the proximal anchors (22) and/or distal anchors (24) comprise looped ends.

3. The prosthesis of Claim 1, wherein all of the plurality of proximal anchors (22) and the plurality of distal anchors (24) comprise looped ends.

4. The prosthesis of any one of Claims 2-3, wherein at least some of the anchors comprising looped ends are connected to the frame (20) at a first base and a second base spaced apart from each other, and wherein adjacent anchors comprising looped ends share a base or strut.

5. The prosthesis of any one of Claims 2-4, wherein at least some of the anchors comprising looped ends are connected to the frame (20) by a single strut.

6. The prosthesis of any one of Claims 1-5, wherein the frame (20), the plurality of proximal anchors (22) and the plurality of distal anchors (24) are formed from a single piece of material.

7. The prosthesis of any one of Claims 1-6, wherein the frame (20) comprises a plurality of foreshortening cells causing a longitudinal length of the frame (20) to decrease as the frame (20) moves from a collapsed size to an expanded size.

8. The prosthesis of any one of Claims 1-7, wherein the proximal anchors (22) and distal anchors (24) are separated by a foreshortening portion of the frame (20).

9. The prosthesis of any one of Claims 1-8, wherein the ends of at least some of the distal anchors (24) are substantially parallel with the longitudinal axis when the frame (20) is at least partially expanded and the proximal and distal anchors (22, 24) are in their expanded configurations.

10. The prosthesis of any one of Claims 1-9, wherein some of the anchors expand to a first radial distance, and some of the anchors expand to a second radial distance, the first radial distance being greater than the second radial distance.

11. The prosthesis of any one of Claims 1-10, wherein at least some of the plurality of proximal anchors (22) expand to a first radial distance, and at least some of the plurality of distal anchors (24) expand to a second radial distance, the first radial distance being greater than the second radial distance.

12. A replacement mitral valve comprising the prosthesis of any one of Claims 1-11, wherein when the frame (20) is in an expanded configuration, the frame (20) is positionable within a native mitral valve annulus such that:
the proximal anchors (22) atraumatically engage tissue on an atrial side of the native mitral valve annulus; and
the distal anchors (24) are positionable within the ventricle and have portions that extend generally proximally toward a ventricular side of the native mitral valve annulus.

13. The replacement mitral valve of Claim 12, wherein the distal anchors (24) are positionable within the ventricle and extend proximally to ends (28) spaced distally from the native mitral valve annulus.

14. The replacement mitral valve of Claim 12, wherein the distal anchors (24) are positionable within the ventricle and extend proximally to ends (28) that engage the native mitral valve annulus.

15. The prosthesis of Claim 1, wherein:
the proximal anchors (22) comprise looped ends which are connected to the frame (20) at a first base (54) and a second base (56) spaced apart from each other, and wherein adjacent anchors comprising looped ends share a base or strut;
the frame (20) comprises a plurality of foreshortening cells causing a longitudinal length of the frame (20) to decrease as the frame (20) moves from a collapsed size to an expanded size; and
between the bases of each proximal anchor (22), the prosthesis comprises a plurality of longitudinally extending struts (80) extending proximally from the frame (20) at a corner of a foreshortening cell, each of the plurality of longitudinally extending struts (80) terminating at their proximal ends in an enlarged portion (8) configured to facilitate holding or retaining the proximal end of the frame (20).

16. The prosthesis of Claim 1, wherein:
the proximal anchors (22) comprise looped ends which are connected to the frame (20) at a first base (54) and a second base (56) spaced apart from each other, and wherein adjacent anchors comprising looped ends share a base or strut; and
between the bases of each proximal anchor (22), the prosthesis comprises a plurality of longitudinally extending struts (80) extending proximally from the frame (20) between bases of each proximal anchor (22), each of the plurality of longitudinally extending struts (80) terminating at their proximal ends in an enlarged portion (8) configured to facilitate holding or retaining the proximal end of the frame (20).

17. The prosthesis of Claim 1, wherein:
the proximal anchors (22) comprise looped ends which are connected to the frame (20) at a first base (54) and a second base (56) spaced apart from each other, and wherein adjacent anchors comprising looped ends share a base or strut;
the frame (20) comprises a plurality of foreshortening cells causing a longitudinal length of the frame (20) to decrease as the frame (20) moves from a collapsed size to an expanded size;
adjacent proximal anchors (22) extend from a same corner of a foreshortening cell.

18. The prosthesis of Claim 1, wherein each of the plurality of proximal anchors (22) is longer than the distal anchors (24) and extends proximally from the frame (20) at bases (54, 56) and is then bent to extend radially outwardly away from the frame (20) in a generally distal direction along a first segment (58) and then bent so that the ends of the proximal anchors (22) become perpendicular to the longitudinal axis of the frame (20).

19. The prosthesis of Claim 1, further comprising an outer skirt (30) having a first portion (64) and a second portion (66), wherein the first portion (64) has a generally cylindrical shape with an inner diameter that substantially corresponds in size to an outer diameter of the frame (20) and the second portion (66) flares outwardly from the first portion (64) and extends generally perpendicularly from the first portion (64).

## Patentansprüche

1. Prothese, gestaltet zum Greifen von intralumenalem Gewebe, wenn in eine Körperhöhle eingesetzt, wobei die Prothese umfasst:
einen expandierbaren Rahmen (20), umfassend ein proximales Ende (32) und ein distales Ende (34) und eine hindurch verlaufende Längsachse, wobei der Rahmen (20) dafür gestaltet ist, zum Einsetzen radial zu kollabieren und nach dem Einsetzen radial zu expandieren, wobei, wenn sich der Rahmen (20) in einer expandierten Konfiguration befindet, der Rahmen (20) in einem mittleren Teil des Rahmens (20) eine größere Querschnittsabmessung und in einem proximalen Teil und einem distalen Teil des Rahmens (20) eine kleinere Querschnittsabmessung aufweist, wobei der mittlere Teil zwischen dem proximalen und dem distalen Teil angeordnet ist;
eine Vielzahl von proximalen Ankern (22), die von dem Rahmen (20) ausgehen und von einer kollabierten Konfiguration zu einer expandierten Konfiguration expandierbar sind, wobei in der expandierten Konfiguration jeder proximale Anker (22) proximal von dem proximalen Ende (32) des Rahmens (20) weg verläuft und sich dann wendet, um distal zu verlaufen, wobei jeder proximale Anker (22) bis zu einem Ende verläuft, das radial außerhalb des Rahmens (20) angeordnet ist; und
eine Vielzahl von distalen Ankern (24), die von dem Rahmen (20) ausgehen und von einer kollabierten Konfiguration zu einer expandierten Konfiguration expandierbar sind, wobei in der expandierten Konfiguration jeder distale Anker (24) distal von dem distalen Ende (34) des Rahmens (20) weg verläuft und sich dann wendet, um proximal zu verlaufen, wobei jeder distale Anker (24) bis zu einem Ende verläuft, das radial außerhalb des Rahmens (20) angeordnet ist;
wobei in der expandierten Konfiguration die Enden von jedem der Vielzahl von proximalen Ankern (22) im Wesentlichen senkrecht zu der Längsachse des Rahmens (20) stehen;
wobei in der expandierten Konfiguration jeder der Vielzahl von distalen Ankern (24) nahe einer Basis des distalen Ankers (24) gebogen ist, um entlang eines ersten Segments (50) von dem Rahmen (20) weg radial nach außen zu verlaufen, und sich dann biegt, um entlang eines zweiten Segments (52), das in einer Spitze (28) endet, allgemein proximal zu zeigen.

2. Prothese gemäß Anspruch 1, wobei wenigstens manche der proximalen Anker (22) und/oder distalen Anker (24) schlaufenförmige Enden umfassen.

3. Prothese gemäß Anspruch 1, wobei alle der Vielzahl von proximalen Ankern (22) und der Vielzahl von distalen Ankern (24) schlaufenförmige Enden umfassen.

4. Prothese gemäß einem der Ansprüche 2-3, wobei wenigstens manche der Anker, die schlaufenförmige Enden umfassen, an einer ersten Basis und einer zweiten Basis, die voneinander beabstandet sind, mit dem Rahmen (20) verbunden sind, und wobei benachbarte Anker, die schlaufenförmige Enden umfassen, eine Basis oder Strebe teilen.

5. Prothese gemäß einem der Ansprüche 2-4, wobei wenigstens manche der Anker, die schlaufenförmige Enden umfassen, durch eine einzelne Strebe mit dem Rahmen (20) verbunden sind.

6. Prothese gemäß einem der Ansprüche 1-5, wobei der Rahmen (20), die Vielzahl von proximalen Ankern (22) und die Vielzahl von distalen Ankern (24) aus einem einzigen Materialstück gebildet sind.

7. Prothese gemäß einem der Ansprüche 1-6, wobei der Rahmen (20) eine Vielzahl von Verkürzungszellen umfasst, die bewirken, dass die Länge des Rahmens (20) in Längsrichtung abnimmt, wenn sich der Rahmen (20) von einer kollabierten Größe zu einer expandierten Größe bewegt.

8. Prothese gemäß einem der Ansprüche 1-7, wobei die proximalen Anker (22) und die distalen Anker (24) durch einen Verkürzungsteil des Rahmens (20) getrennt sind.

9. Prothese gemäß einem der Ansprüche 1-8, wobei die Enden wenigstens mancher der distalen Anker (24) im Wesentlichen parallel zu der Längsachse sind, wenn der Rahmen (20) wenigstens teilweise expandiert ist und die proximalen und die distalen Anker (22, 24) in ihren expandierten Konfigurationen vorliegen.

10. Prothese gemäß einem der Ansprüche 1-9, wobei manche der Anker auf einen ersten radialen Abstand expandieren und manche der Anker auf einen zweiten radialen Abstand expandieren, wobei der erste radiale Abstand größer als der zweite radiale Abstand ist.

11. Prothese gemäß einem der Ansprüche 1-10, wobei wenigstens manche der Vielzahl von proximalen Ankern (22) auf einen ersten radialen Abstand expandieren und wenigstens manche der Vielzahl von distalen Ankern (24) auf einen zweiten radialen Abstand expandieren, wobei der erste radiale Abstand größer als der zweite radiale Abstand ist.

12. Ersatz-Mitralklappe, umfassend eine Prothese gemäß einem der Ansprüche 1-11, wobei, wenn sich der Rahmen (20) in einer expandierten Konfiguration befindet, der Rahmen (20) innerhalb eines nativen Mitralklappenrings positionierbar ist, so dass:
die proximalen Anker (22) atraumatisch mit Gewebe an einer atrialen Seite des nativen Mitralklappenrings in Eingriff stehen; und
die distalen Anker (24) innerhalb des Ventrikels positionierbar sind und Teile aufweisen, die allgemein proximal in Richtung zu einer vetrikulären Seite des nativen Mitralklappenrings verlaufen.

13. Ersatz-Mitralklappe gemäß Anspruch 12, wobei die distalen Anker (24) innerhalb des Ventrikels positionierbar sind und proximal zu Enden (28) verlaufen, die distal von dem nativen Mitralklappenring beabstandet sind.

14. Ersatz-Mitralklappe gemäß Anspruch 12, wobei die distalen Anker (24) innerhalb des Ventrikels positionierbar sind und proximal zu Enden (28) verlaufen, die mit dem nativen Mitralklappenring in Eingriff stehen.

15. Prothese gemäß Anspruch 1, wobei:
die proximalen Anker (22) schlaufenförmige Enden umfassen, die an einer ersten Basis (54) und einer zweiten Basis (56), die voneinander beabstandet sind, mit dem Rahmen (20) verbunden sind, und wobei benachbarte Anker, die schlaufenförmige Enden umfassen, eine Basis oder Strebe teilen;
der Rahmen (20) eine Vielzahl von Verkürzungszellen umfasst, die bewirken, dass die Länge des Rahmens (20) in Längsrichtung abnimmt, wenn sich der Rahmen (20) von einer kollabierten Größe zu einer expandierten Größe bewegt; und
die Prothese zwischen den Basen jedes proximalen Ankers (22) eine Vielzahl von in Längsrichtung verlaufenden Streben (80) umfasst, die von dem Rahmen (20) proximal an einer Ecke der Verkürzungszelle ausgehen, wobei jede der Vielzahl von in Längsrichtung verlaufenden Streben (80) an ihren proximalen Enden in einem vergrößerten Teil (8) endet, der dafür gestaltet ist, Halten oder Zurückhalten des proximalen Endes des Rahmens (20) zu erleichtern.

16. Prothese gemäß Anspruch 1, wobei:
die proximalen Anker (22) schlaufenförmige Enden umfassen, die an einer ersten Basis (54) und einer zweiten Basis (56), die voneinander beabstandet sind, mit dem Rahmen (20) verbunden sind, und wobei benachbarte Anker, die schlaufenförmige Enden umfassen, eine Basis oder Strebe teilen; und
die Prothese zwischen den Basen jedes proximalen Ankers (22) eine Vielzahl von in Längsrichtung verlaufenden Streben (80) umfasst, die von dem Rahmen (20) proximal zwischen Basen jedes proximalen Ankers (22) ausgehen, wobei jede der Vielzahl von in Längsrichtung verlaufenden Streben (80) an ihren proximalen Enden in einem vergrößerten Teil (8) endet, der dafür gestaltet ist, Halten oder Zurückhalten des proximalen Endes des Rahmens (20) zu erleichtern.

17. Prothese gemäß Anspruch 1, wobei:
die proximalen Anker (22) schlaufenförmige Enden umfassen, die an einer ersten Basis (54) und einer zweiten Basis (56), die voneinander beabstandet sind, mit dem Rahmen (20) verbunden sind, und wobei benachbarte Anker, die schlaufenförmige Enden umfassen, eine Basis oder Strebe teilen;
der Rahmen (20) eine Vielzahl von Verkürzungszellen umfasst, die bewirken, dass die Länge des Rahmens (20) in Längsrichtung abnimmt, wenn sich der Rahmen (20) von einer kollabierten Größe zu einer expandierten Größe bewegt;
benachbarte proximale Anker (22) von einer gleichen Ecke einer Verkürzungszelle ausgehen.

18. Prothese gemäß Anspruch 1, wobei jeder der Vielzahl von proximalen Ankern (22) länger als die distalen Anker (24) ist und von dem Rahmen (20) proximal an Basen (54, 56) ausgeht und sich dann biegt, um in einer allgemein distalen Richtung entlang eines ersten Segments (58) von dem Rahmen (20) weg radial nach außen zu verlaufen, und sich dann biegt, so dass die Enden der proximalen Anker (22) senkrecht zu der Längsachse des Rahmens (20) werden.

19. Prothese gemäß Anspruch 1, ferner umfassend eine äußere Schürze (30), die einen ersten Teil (64) und einen zweiten Teil (66) aufweist, wobei der erste Teil (64) eine allgemein zylindrische Form mit einem Innendurchmesser aufweist, der in seiner Größe im Wesentlichen einem Außendurchmesser des Rahmens (20) entspricht, und der zweite Teil (66) von dem ersten Teil (64) nach außen aufgeweitet ist und im Wesentlichen senkrecht zu dem ersten Teil (64) verläuft.

## Revendications

1. Prothèse configurée pour saisir un tissu intracavitaire lorsqu'elle est déployée dans une cavité corporelle, la prothèse comprenant :
un cadre pouvant se déployer (20) comprenant une extrémité proximale (32) et une extrémité distale (34), et un axe longitudinal s'étendant à travers celui-ci, le cadre (20) étant configuré pour se replier radialement pour la pose et pour se déplier radialement lors du déploiement, lorsque le cadre (20) est dans une configuration dépliée, le cadre (20) ayant une plus grande dimension transversale dans une partie centrale du cadre (20) et une plus petite dimension transversale dans une partie proximale et une partie distale du cadre (20), la partie centrale étant entre les parties proximale et distale ;
une pluralité d'éléments d'ancrage proximaux (22) s'étendant à partir du cadre (20) et pouvant se déployer depuis une configuration repliée vers une configuration dépliée, dans la configuration dépliée, chaque élément d'ancrage proximal (22) s'étendant de manière proximale en s'éloignant de l'extrémité proximale (32) du cadre (20) et en se tournant ensuite pour s'étendre de manière distale, chaque élément d'ancrage proximal (22) s'étendant jusqu'à une extrémité positionnée de manière radiale vers l'extérieur à partir du cadre (20) ; et
une pluralité d'éléments d'ancrage distaux (24) s'étendant à partir du cadre (20) et pouvant se déployer depuis une configuration repliée vers une configuration dépliée, dans la configuration dépliée, chaque élément d'ancrage distal (24) s'étendant de manière distale en s'éloignant de l'extrémité distale (34) du cadre (20) et en se tournant ensuite pour s'étendre de manière proximale, chaque élément d'ancrage distal (24) s'étendant jusqu'à une extrémité positionnée de manière radiale vers l'extérieur à partir du cadre (20) ;
dans la configuration dépliée, les extrémités de chacun de la pluralité d'éléments d'ancrage proximaux (22) étant sensiblement perpendiculaires à l'axe longitudinal du cadre (20) ;
dans la configuration dépliée, chacun de la pluralité d'éléments d'ancrage distaux (24) étant courbé près d'une base de l'élément d'ancrage distal (24) pour s'étendre de manière radiale vers l'extérieur en s'éloignant du cadre (20) le long d'un premier segment (50) et étant ensuite courbé pour pointer généralement de manière proximale le long d'un second segment (52) se terminant par une pointe (28).

2. Prothèse selon la revendication 1, au moins une partie des éléments d'ancrage proximaux (22) et/ou des éléments d'ancrage distaux (24) comprenant des extrémités en boucle.

3. Prothèse selon la revendication 1, la totalité de la pluralité d'éléments d'ancrage proximaux (22) et la pluralité d'éléments d'ancrage distaux (24) comprenant des extrémités en boucle.

4. Prothèse selon l'une quelconque des revendications 2 et 3, au moins certains des ancrages qui comprennent des extrémités en boucle étant reliés au cadre (20) au niveau d'une première base et au niveau d'une seconde base espacées l'une de l'autre, et des éléments d'ancrage adjacents qui comprennent des extrémités en boucle partageant une base ou une entretoise.

5. Prothèse selon l'une quelconque des revendications 2 à 4, au moins certains des éléments d'ancrage qui comprennent des extrémités en boucle étant reliés au cadre (20) par une seule entretoise.

6. Prothèse selon l'une quelconque des revendications 1 à 5, le cadre (20), la pluralité d'éléments d'ancrage proximaux (22) et la pluralité d'éléments d'ancrage distaux (24) étant formés à partir d'une seule pièce de matériau.

7. Prothèse selon l'une quelconque des revendications 1 à 6, le cadre (20) comprenant une pluralité de cellules de raccourcissement amenant une longueur longitudinale du cadre à diminuer (20) au fur et à mesure que le cadre (20) passe d'une dimension repliée à une dimension dépliée.

8. Prothèse selon l'une quelconque des revendications 1 à 7, les éléments d'ancrage proximaux (22) et les éléments d'ancrage distaux (24) étant séparés par une partie de raccourcissement du cadre (20).

9. Prothèse selon l'une quelconque des revendications 1 à 8, les extrémités d'au moins certains des éléments d'ancrage distaux (24) étant sensiblement parallèles à l'axe longitudinal lorsque le cadre (20) est au moins partiellement déplié et que les éléments d'ancrage proximaux et distaux (22, 24) sont dans leurs configurations dépliées.

10. Prothèse selon l'une quelconque des revendications 1 à 9, certains des éléments d'ancrage se dépliant jusqu'à une première distance radiale, et certains des éléments d'ancrage se dépliant jusqu'à une seconde distance radiale, la première distance radiale étant supérieure à la seconde distance radiale.

11. Prothèse selon l'une quelconque des revendications 1 à 10, au moins certains de la pluralité d'éléments d'ancrage proximaux (22) se dépliant jusqu'à une première distance radiale, et au moins certains de la pluralité d'éléments d'ancrage distaux (24) se dépliant jusqu'à une seconde distance radiale, la première distance radiale étant supérieure à la seconde distance radiale.

12. Valvule mitrale de remplacement comprenant la prothèse selon l'une quelconque des revendications 1 à 11, lorsque le cadre (20) est dans une configuration dépliée, le cadre (20) pouvant être positionné à l'intérieur d'un anneau de valvule mitrale native de telle sorte que :
les éléments d'ancrage proximaux (22) viennent en prise de manière atraumatique avec le tissu sur un côté auriculaire de l'anneau de valvule mitrale native ; et
les éléments d'ancrage distaux (24) peuvent être positionné dans le ventricule et ont des parties qui s'étendent généralement de manière proximale vers un côté ventriculaire de l'anneau de valvule mitrale native.

13. Valvule mitrale de remplacement selon la revendication 12, les éléments d'ancrage distaux (24) pouvant être positionnés à l'intérieur du ventricule et s'étendant de manière proximale aux extrémités (28) espacées de manière distale de l'anneau de valvule mitrale native.

14. Valvule mitrale de remplacement selon la revendication 12, les éléments d'ancrage distaux (24) pouvant être positionnés à l'intérieur du ventricule et s'étendant de manière proximale aux extrémités (28) qui viennent en prise avec l'anneau de valvule mitrale native.

15. Prothèse selon la revendication 1,
les éléments d'ancrage proximaux (22) comprenant des extrémités en boucle qui sont reliées au cadre (20) au niveau d'une première base (54) et d'une seconde base (56) espacées l'une de l'autre, et des éléments d'ancrage adjacents comprenant des extrémités en boucle partageant une base ou une entretoise ;
le cadre (20) comprenant une pluralité de cellules de raccourcissement amenant une longueur longitudinale du cadre (20) à diminuer au fur et à mesure que le cadre (20) passe d'une dimension repliée à une dimension dépliée ; et
entre les bases de chaque élément d'ancrage proximal (22), la prothèse comprenant une pluralité d'entretoises s'étendant de manière longitudinale (80), s'étendant de manière proximale à partir du cadre (20) au niveau d'un coin d'une cellule de raccourcissement, chacune de la pluralité d'entretoises s'étendant de manière longitudinale (80), se terminant au niveau de leurs extrémités proximales dans une partie élargie (8) configurée pour faciliter le maintien ou la retenue de l'extrémité proximale du cadre (20).

16. Prothèse selon la revendication 1,
les éléments d'ancrage proximaux (22) comprenant des extrémités en boucle qui sont reliés au cadre (20) au niveau d'une première base (54) et d'une seconde base (56) espacées l'une de l'autre, et des éléments d'ancrage adjacents comprenant des extrémités en boucle partageant une base ou une entretoise ; et
entre les bases de chaque élément d'ancrage proximal (22), la prothèse comprenant une pluralité d'entretoises s'étendant de manière longitudinale (80) s'étendant de manière proximale à partir du cadre (20) entre les bases de chaque élément d'ancrage proximal (22), chacune de la pluralité d'entretoises s'étendant de manière longitudinale (80) se terminant au niveau de leurs extrémités proximales en une partie élargie (8) configurée pour faciliter le maintien ou la retenue de l'extrémité proximale du cadre (20).

17. Prothèse selon la revendication 1,
les éléments d'ancrage proximaux (22) comprenant des extrémités en boucle qui sont reliées au cadre (20) au niveau d'une première base (54) et d'une seconde base (56) espacées l'une de l'autre, et des éléments d'ancrage adjacents comprenant des extrémités en boucle partageant une base ou une entretoise ;
le cadre (20) comprenant une pluralité de cellules de raccourcissement amenant une longueur longitudinale du cadre (20) à diminuer au fur et à mesure que le cadre (20) passe d'une dimension repliée à une dimension dépliée ;
des éléments d'ancrage proximaux adjacents (22) s'étendant à partir d'un même coin d'une cellule de raccourcissement.

18. Prothèse selon la revendication 1, chacun de la pluralité d'éléments d'ancrage proximaux (22) étant plus long que les éléments d'ancrage distaux (24) et s'étendant de manière proximale à partir du cadre (20) au niveau des bases (54, 56) et étant ensuite courbé pour s'étendre de manière radiale vers l'extérieur à partir du cadre (20) dans une direction généralement distale le long d'un premier segment (58) et ensuite courbé de sorte que les extrémités des éléments d'ancrage proximaux (22) deviennent perpendiculaires à l'axe longitudinal du cadre (20) .

19. Prothèse selon la revendication 1, comprenant en outre une jupe extérieure (30) ayant une première partie (64) et une seconde partie (66), la première partie (64) ayant une forme généralement cylindrique avec un diamètre intérieur qui correspond sensiblement en dimension à un diamètre extérieur du cadre (20) et la seconde partie (66) s'évasant vers l'extérieur à partir de la première partie (64) et s'étendant généralement perpendiculairement à partir de la première partie (64).
